(19) Europäisches Patentamt
European Patent Office
Office européen des brevets

(11) Veröffentlichungsnummer: **0 251 194 B1**

# (12) EUROPÄISCHE PATENTSCHRIFT

(45) Veröffentlichungstag der Patentschrift: **31.03.93**

(21) Anmeldenummer: **87109110.4**

(22) Anmeldetag: **24.06.87**

(51) Int. Cl.5: **C07D 217/18**, C07D 491/056, C07D 471/04, C07D 495/04, C07D 295/18, A61K 31/47, A61K 31/395

## (54) Verwendung carbocyclisch und heterocyclisch anellierter Dihydropyridine zur Herstellung von cardioprotektiven Mitteln sowie neue heterocyclisch und carbocyclisch anellierte Dihydropyridine, Verfahren zu deren Herstellung und Zwischenstufen für deren Herstellung.

(30) Priorität: **26.06.86 DE 3621413**

(43) Veröffentlichungstag der Anmeldung:
**07.01.88 Patentblatt 88/01**

(45) Bekanntmachung des Hinweises auf die Patenterteilung:
**31.03.93 Patentblatt 93/13**

(84) Benannte Vertragsstaaten:
**AT BE CH DE ES FR GB GR IT LI LU NL SE**

(56) Entgegenhaltungen:
**EP-A- 0 037 934**
**EP-A- 0 101 574**
**EP-B- 0 003 920**

(73) Patentinhaber: **BOEHRINGER INGELHEIM KG**
**Postfach 200**
**W-6507 Ingelheim am Rhein(DE)**
(84) Benannte Vertragsstaaten:
**AT BE CH DE ES FR GB GR IT LI LU NL SE**

(73) Patentinhaber: **BOEHRINGER INGELHEIM INTERNATIONAL GmbH**
**Postfach 20**
**W-6507 Ingelheim am Rhein(DE)**
(84) Benannte Vertragsstaaten:
**GB**

(72) Erfinder: **Lösel, Walter, Dr.**
**Im Herzenacker 26**
**W-6535 Gau-Algesheim(DE)**
Erfinder: **Schnorrenberg, Gerd, Dr.**
**Ernst-Ludwig-Strasse 66a**
**W-6535 Gau-Algesheim(DE)**
Erfinder: **Roos, Otto, Dr.**
**Elsheimer Strasse 36**
**W-6501 Schwabenheim(DE)**
Erfinder: **Arndts, Dietrich, Dr.**
**Mühlstrasse 7**
**W-6531 Appenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

Erfinder: **Streller, Ilse, Dr.**
**Waldstrasse 16**
**W-6534 Stromberg(DE)**
Erfinder: **Traunecker, Werner, Dr.**
**Birkenweg 1**
**W-6538 Münster-Sarmsheim(DE)**

## Beschreibung

Aus der EP-A 37 934 sind Dihydroisochinoline der allgemeinen Formel Ia bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden.

Es wurde nun überraschend gefunden, daß die durch die allgemeinen Formeln I und II beschriebenen Substanzen eine hervorragende cardioprotektive Wirkung besitzen und einen völlig neuen Typ Ca-antagonistischer Verbindungen verkörpern.

Die Erfindung betrifft somit die Verwendung von carbocyclisch und heterocyclisch anellierten Dihydropyridinen der Formel

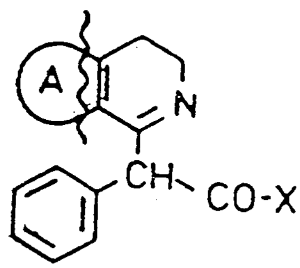

I

sowie deren tautomere Formen der Formel II, wobei diese Bezeichnung zusammenfassend für die cis- und trans-Formen II' und II'' steht:

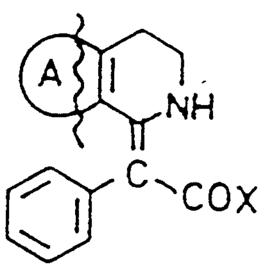

oder

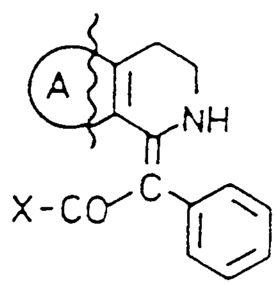

II' II''

II

in der X für $OR_1$; $NHR_2$; $NR_3R_4$
und

$R_1$ für Wasserstoff, $C_1$-$C_4$-Alkyl; Alkoxyalkyl mit bis 4 C-Atomen;

$R_2$ für Wasserstoff; $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c), die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können)

b) Phenyl; gewünschtenfalls ein- oder mehrfach gleich oder verschieden substituiert durch die Gruppen Halogen, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe -O-$(CH_2)_n$-O (mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S; sowie als bicylischer Heterocyclus Indol (wobei die zuvor genannen Heterocyclen ein- oder mehrfach durch $C_1$-$C_4$-Alkyl substituiert sein können, $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann).

$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann.

oder

$R_3$ und $R_4$ zusammen mit dem Stickstoffatom, an welches sie gebunden sind für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0,1) substituiert sein kann

und

$R_5$ für ein Phenylradikal, welches gewünschtenfalls wie vorstehend unter b) substituiert ist, steht;

A steht für die anellierten Ringsysteme

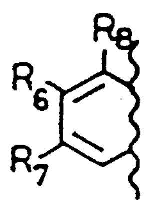
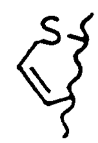
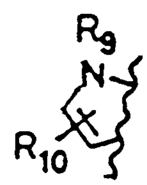
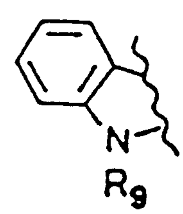

in denen

$R_8$ für Wasserstoff; $C_1$-$C_4$-Alkyl; oder $C_1$-$C_4$-Alkoxy

$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für $-O-(CH_2)_n-O-$ (mit n = 1,2)

$R_9$ für Wasserstoff, $C_1$-$C_4$-Alkyl

$R_{10}$ für Wasserstoff; oder 2-Phenyl-2-ethoxycarbonylacetyl

steht

und von Verbindungen der allgemeinen Formel I bzw. II

in der

X für $NHR_2$,

$R_2$ für 3-Chlorphenyl oder 2-Methyl-3-chlorphenyl;

und

A für die anellierten Ringsysteme

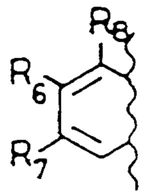

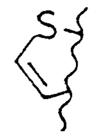

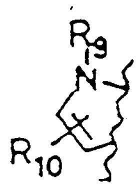

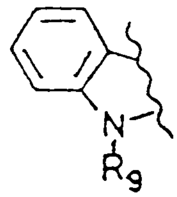

in denen

$R_8$ für Wasserstoff, oder Methoxy;
$R_6$ für Methoxy, Hydroxy, Amino oder Methansulfonylamino;
$R_7$ für Wasserstoff, Methoxy, oder Hydroxy;
$R_9$ für Methyl;
$R_{10}$ für 2-Phenyl-2-ethoxycarbonylacetyl

steht;

sowie deren Salze mit physiologisch verträglichen Säuren, Basen oder Komplexbildnern zur Cardioprotektion.

Bevorzugt ist die Verwendung von carbocyclisch und heterocyclisch anellierten Dihydropyridinen der Formel

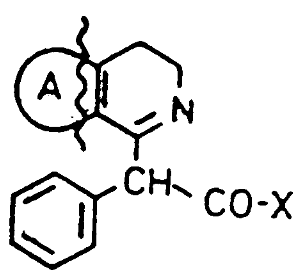

I

sowie deren tautomere Formen der Formel

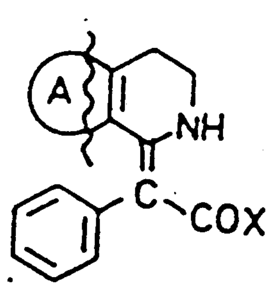

oder

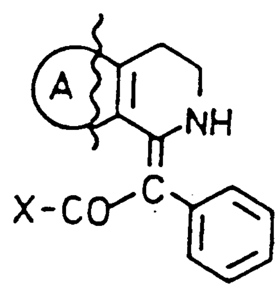

II'

II''

II

in der X für $OR_1$ $NHR_2$; $NR_3R_4$

$R_1$ für Methyl oder Ethyl

$R_2$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;

d) Cyano, Hydroxy, Methoxy, Dimethylamino

e) Phenyl, 3,4-Methylendioxyphenyl, 2,4-Dimethoxyphenyl, 4-Methoxyphenyl, 3,4-Dimethoxyphenyl, 2-Methoxyphenyl, 3-Methoxy-4-hydroxyphenyl, 3,4,5-Trimethoxyphenyl, 3-Hydroxy-4-methoxyphenyl,

f) Morpholino, Pyridin-2-yl, Indol-3-yl, Furan-2-yl, Thiophen-2-yl, Pyridin-3-yl, Pyridin-4-yl

$R_3$ und $R_4$ unabhängig voneinander für Methyl; Ethyl; 3-Cyanopropyl; Benzyl; oder 3,4,5-Trimethoxyphenethyl stehen oder

$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;

und

A für die anellierten Ringsysteme

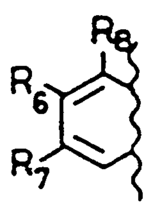

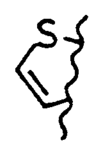

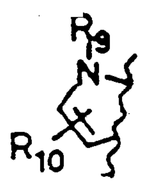

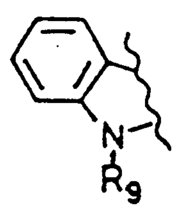

in denen

$R_8$ für Wasserstoff; oder Methoxy

$R_6$ für Methoxy; Hydroxy; Wasserstoff; Amino; oder Methansulfonylamino

$R_7$ für Wasserstoff; Methoxy; oder Hydroxy;

$R_9$ für Methyl;

$R_{10}$ für 2-Phenyl-2-ethoxycarbonylacetyl; oder Wasserstoff;

steht, sowie deren Salze mit physiologisch verträglichen Säuren zur Cardioprotektion.

Die Erfindung betrifft somit Dihydroisochinoline der Formel

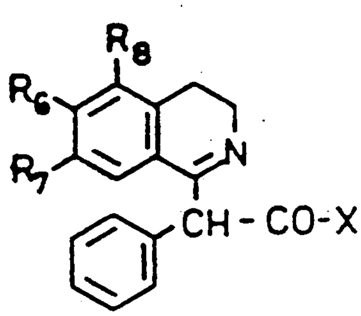

I a

Dihydro-thieno[3,2-c]pyridine der Formel

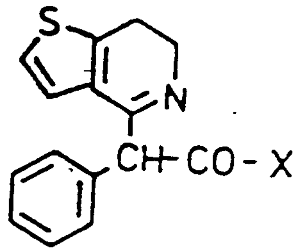

Ib

Dihydro-pyrollo[3,2-c]pyridine der Formel

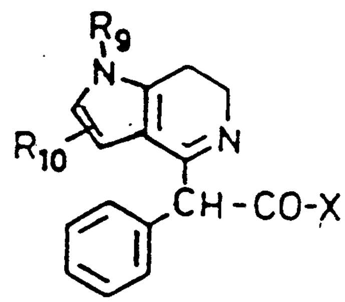

Ic

und Dihydro-pyrido[3,4-b]indole der Formel

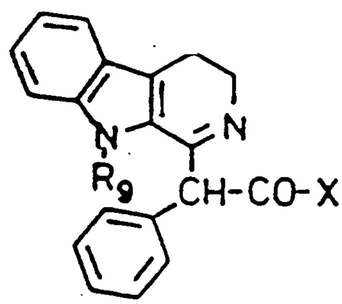

Id

sowie deren tautomere Formen IIa', IIb', IIc' und IId' und die dazu E/Z-isomeren Formen IIa'', IIb'', IIc'' und IId''.

Die Verbindungen der Formel Ia - Id sind chiral. Die Erfindung umfaßt auch die beiden R- und S-enantiomeren Formen der Verbindungen der Formel I bei denen die Bedeutung der Reste X, $R_6$, $R_7$, $R_8$, $R_9$ und $R_{10}$ wie zuvor definiert ist.

Bevorzugt werden Verbindungen der Formel I bzw. II verwendet, worin A der Rest

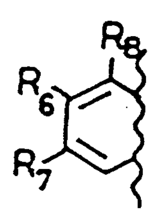

ist (insbesondere solche, worin $R_8$ Wasserstoff ist und $R_6$ und $R_7$ unabhängig voneinander Hydroxy oder Methoxy oder gemeinsam -O-$CH_2$-O-sind) und solche worin X -$NHR_2$ oder -$NR_3R_4$ ist, (insbesondere solche, worin X-$NHR_2$ ist, worin $R_2$

$C_1$-$C_6$-alkyl ist, vorzugsweise $C_1$-$C_4$-Alkyl, das unsubstituiert ist oder durch Hydroxy, Phenyl (das durch Hydroxy, Methoxy oder -O-$CH_2$-O- substituiert sein kann) oder Morpholino substituiert ist).

Die in den nachstehenden Tabellen 1 bis 11 aufgeführten Substanzen liegen jeweils bevorzugt in der tautomeren Form I oder II vor.

Die bevorzugte tautomere Form ist in der Spalte Struktur mit I bzw. II gekennzeichnet.

Namentlich zu nennen sind folgende Verbindungen der Formel I in der tautomeren Form I bzw. II.

Tabelle 1

Strukturtyp:

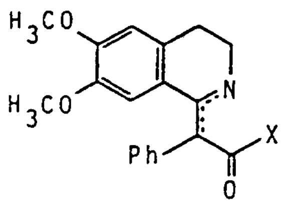

| Nr. | X | Struktur |
|---|---|---|
| 1. | $OCH_3$ | II |
| 2. | $OC_2H_5$ | II |
| 3. | $NHCH_3$ | I |
| 4. | $NHC_2H_5$ | I |
| 5. | $NH-(CH_2)_2-CH_3$ | I |
| 6. | $NH-(CH_2)_3-CH_3$ | I |
| 7. | $NH-(CH_2)_4-CH_3$ | I |
| 8. | $NH-CH(CH_3)_2$ | I |
| 9. | $NH-CH_2-CH(CH_3)_2$ | I |
| 10. | $NH-(CH_2)_2-CH(CH_3)_2$ | I |
| 11. | $NH-C(CH_3)_3$ | I |
| 12. | $NH-CH(CH_3)-C_2H_5$ | I |
| 13. | $NH-CH_2-CH=CH_2$ | I |
| 14. | $NH-CH_2-C\equiv CH$ | I |
| 15. | $NH-(CH_2)_2-OH$ | II |
| 16. | $NH-CH_2-CH(OH)-CH_3$ | I |
| 17. | $NH-(CH_2)_2-OCH_3$ | II |
| 18. | $NH-(CH_2)_3-OCH_3$ | II |
| 19. | $NH-(CH_2)_2-N(CH_3)_2$ | I |
| 20. | $NH-(CH_2)_3-N(CH_3)_2$ | II |
| 21. | $NH-(CH_2)_2-$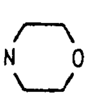 | II |

| Nr. | X | Struktur |
|---|---|---|
| 22. | 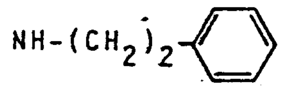 | I |
| 23. | 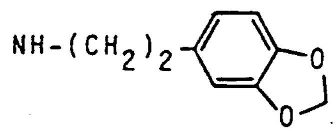 | I |
| 24. | 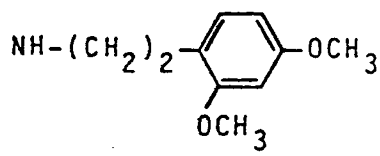 | I |
| 25. | 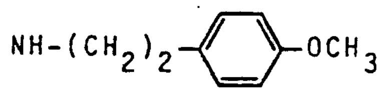 | I |
| 26. | 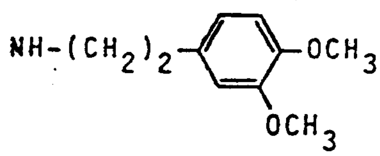 | II |
| 27. | 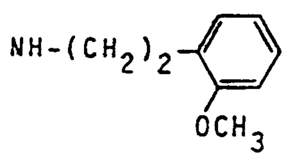 | II |
| 28. | 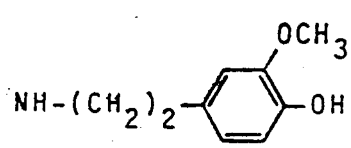 | I |
| 29. | 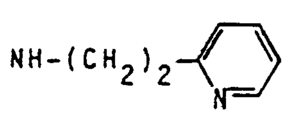 | I |

| Nr. | X | Struktur |
|---|---|---|
| 30. | 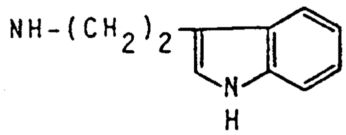 | II |
| 31. | 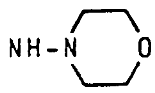 | I |
| 32. | 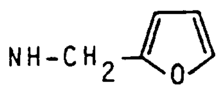 | II |
| 33. | 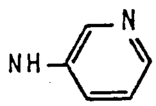 | I |
| 34. | 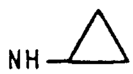 | I |
| 35. | 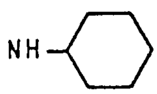 | II |
| 36. | $N(CH_3)_2$ | I |
| 37. | $N(C_2H_5)_2$ | I |
| 38. | 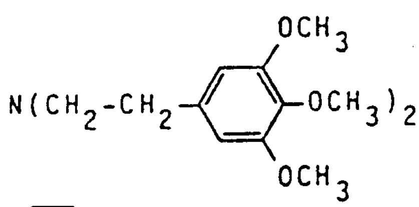 | I |
| 39. | 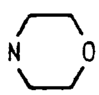 | I |

| Nr. | X | Struktur |
|---|---|---|
| 40 | 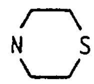 | I |
| 41. | 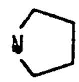 | I<br>II |
| 42. | 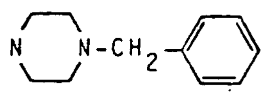 | II |
| 43 | 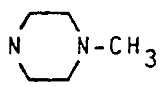 | I |
| 44 | 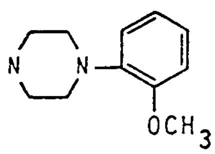 | I |

Tabelle 2

Strukturtyp:

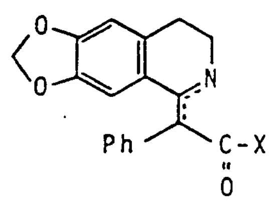

| Nr. | X | Struktur |
|---|---|---|
| 45. | $OC_2H_5$ | II |
| 46. | $NH-(CH_2)_2-$ 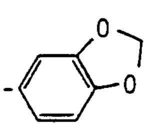 | II |
| 47. | $NH-(CH_2)_2$ 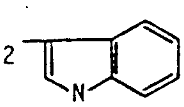 | II |

| Nr. | X | Struktur |
|---|---|---|
| 48. | $NH-(CH_2)_2-$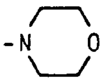 | II |
| 49. | 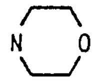 | II |
| 50. | 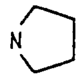 | I |

Tabelle 3

Strukturtyp:

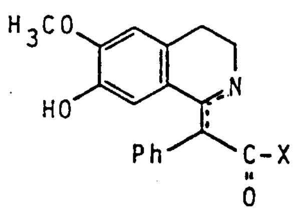

| Nr. | X | Struktur |
|---|---|---|
| 51. | $OC_2H_5$ | I<br>II |
| 52. | $NH-(CH_2)_3-CH_3$ | I |
| 53. | $NH-(CH_2)_4-CH_3$ | I |
| 54. | $NH-CH(CH_3)_2$ | I |
| 55. | $NH-CH_2-CH(CH_3)_2$ | I |
| 56. | $NH-CH_2-CH=CH_2$ | I |
| 57. | $NH-(CH_2)_2-$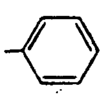 | I |
| 58. | $NH-(CH_2)_2-$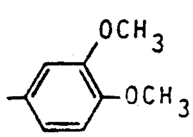 | I |

| Nr. | X | Struktur |
|---|---|---|
| 59. | $NH-(CH_2)_2-$ 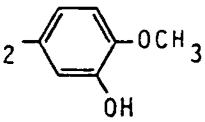 | I |
| 60. | $NH-(CH_2)_2-$ 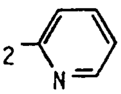 | I |
| 61. | $NH-(CH_2)_2-$ 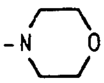 | I |
| 62. | 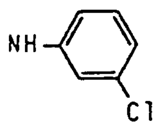 | I |
| 63. | 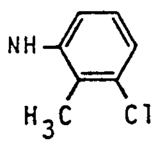 | I |
| 64. | 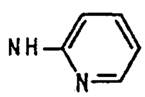 | I |
| 65. | 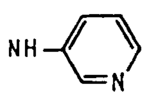 | I |
| 66. | 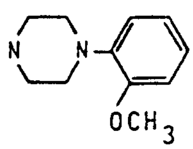 | I |
| 67. | 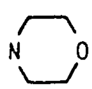 | I |
| 68. | 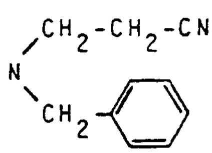 | I |

Tabelle 4

Strukturtyp:

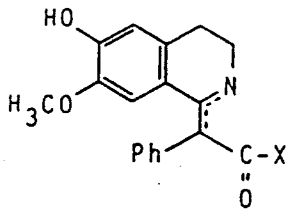

| Nr. | X | Struktur |
| --- | --- | --- |
| 69. | $N(C_2H_5)_2$ | I |

Tabelle 5

Strukturtyp:

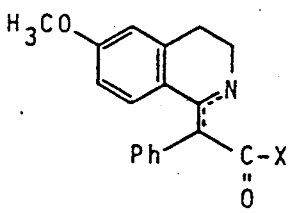

| Nr. | X | Struktur |
| --- | --- | --- |
| 70. | $NHCH_3$ | I |
| 71. | $NHC_2H_5$ | I |
| 72. | $NH-CH_2-$ 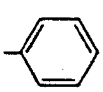 | I |
| 73. | $NH(CH_2)_2-$ 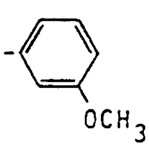 | I |
| 74. | $N(CH_3)C_2H_5$ | I |

Tabelle 6

Strukturtyp:

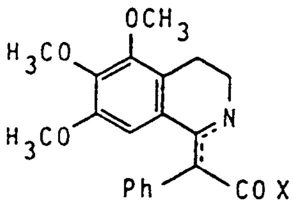

| Nr. | X | Struktur |
|---|---|---|
| 75. | 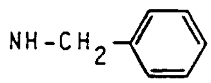 | I |
| 76. | 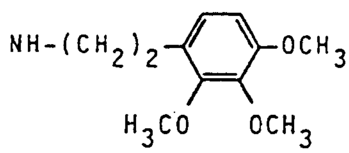 | I |
| 77. | $N(CH_3)C_2H_5$ | I |
| 78. | 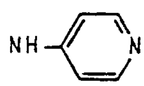 | II |
| 79. | 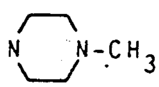 | I |

Tabelle 7

Strukturtyp:

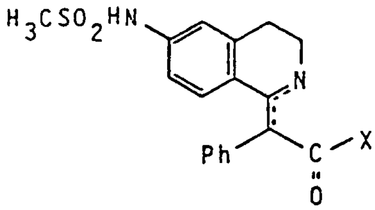

| Nr. | X | Struktur |
|---|---|---|
| 80 | $OC_2H_5$ | II |

Tabelle 8

Strukturtyp:

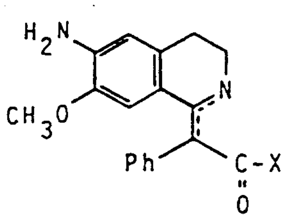

| Nr. | X | Struktur |
|---|---|---|
| 81. | $OC_2H_5$ | II |

Tabelle 9

Strukturtyp:

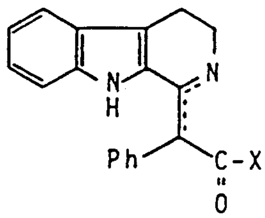

| Nr. | X | Struktur |
|---|---|---|
| 82. | $OC_2H_5$ | I<br>II |
| 83. | $NH-(CH_2)_2-$ 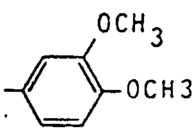 | II |
| 84. | $NH-(CH_2)_2-$ 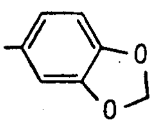 | II |
| 85. | $NH-(CH_2)_2-$ 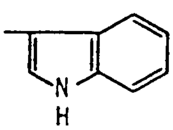 | I<br>II |
| 86. | 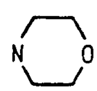 | I |
| 87. | $NH-CH_2-CH(CH_3)_2$ | II |
| 88. | $NH-(CH_2)_3-N(CH_3)_2$ | II |
| 89. | $NH-(CH_2)_2-$ 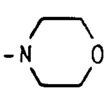 | II |

Tabelle 10

Strukturtyp:

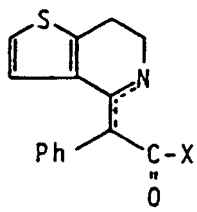

| Nr. | X | Struktur |
|---|---|---|
| 90. | $OC_2H_5$ | |
| 91. | NH-$(CH_2)_2$-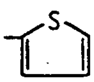 | II |

Tabelle 11

Strukturtyp:

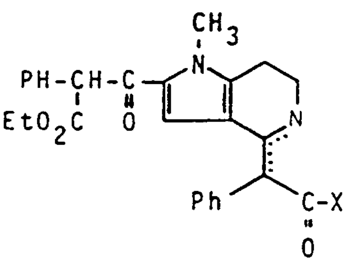

| Nr. | X | Struktur |
|---|---|---|
| 92. | $OC_2H_5$ | II |

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind $C_1$-$C_6$-Alkyl und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl-, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Die vorstehende Definition gilt somit auch wenn das Alkylradikal seinerseits substituiert und/oder selbst Bestandteil einer Alkoxyalkyl-, Alkoxycarbonyl-, Alkoxy-, Alkylthio-, Alkylsulfonyl-, Monoalkylamino-, Alkylmethyl-, Alkylthiomethyl-, Dialkylaminogruppe ist oder das Alkylradikal als Substituent an ein aromatisches heterocyclisches oder carboxyclisches System gebunden ist.

Halogene sind Fluor, Chlor, Brom und Jod, vorzugsweise Fluor, Chlor und Brom und in zweiter Linie Jod.

$C_3$-$C_6$-Cycloalkyl sind Cyclopropan, Cyclobutan, Cyclopentan und Cyclohexan.

$C_5$-$C_6$-Cycloalkene sind z.B. Cyclopenten, Cyclohexen und Cyclohexadien.

Das $C_2$- und $C_3$-Acylradikal steht für das Acetyl und das Propionylradikal.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl

Als ungesättigte Heterocyclen sind unter anderem zu nennen: Furan, Pyran, Pyrrol, Pyrazol, Imidazol, Pyridin, Pyrazin, Pyrimidin, Pyridazin, Thiophen, Thiazol, Oxazol, 1,2,4-Triazol, 1,2,3-Triazol, 1,2,4-Triazin, 1,3,5-Triazin, Indol.

Als 5- oder 6-gliedrige ganz oder teilweise gesättigte monocyclische Heterocyclen sind unter anderem zu nennen:

Imidazolidin Pyrazolidin, Pyrrolidin, Piperidin, Piperazin, Morpholin, Thiomorpholin, Tetrahydrofuran, Tetrahydrothiophen, 1,4-Dioxin,Imidazolin, Pyrazolin, Pyrrolin, etc.

Die erfindungsgemäßen Verbindungen der Formel I bzw. II sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Es wurde nun überraschenderweise gefunden, daß Verbindungen der Formel I bzw. II cardioprotektiv wirksam sind.

Die cadioprotektive Wirkung wird auf folgende Weise bestimmt:

Bekanntlich ist der myocardiale $Ca^{+2}$ -Gehalt ein Maß für die hypoxische bzw. die durch toxische Catecholamindosen hervorgerufene Herzschädigung (Higgins et al., Mol. Cell. Cardiol. 10: 427 - 438, 1984; Nakanishi et al., Am. J. Physiol. 242: 437 - 449, 1982; Fleckenstein, A., Vorträge der Erlanger Physiol. Tagung 1970, Ed. Keidel, Springer Verlag Berlin, Heidelberg, New York, 1971). Umgekehrt ist die Inhibition der hypoxischen oder Isoprenalin-bedingten mycardialen Calciumaufnahme ein Maß für die cardioprotektive Effektivität von Calciumantagonisten (Fleckenstein s.o.), von Calmodulininhibitoren (Higgins) und anderen Pharmaka, z.B. Betaadrenolytika (Arndts, Arzneimittelforschung 25: 1279 - 1284, 1975).

Die cardioprotektive Wirkung wurde an wachen Ratten nach subcutaner oder peroraler Wirkstoffgabe anhand der von Arndts (s.o.) beschriebenen Methode festgestellt, und die Wirkungsstärke der Testsubtanzen als $H_{50}$-Wert angegeben; dieser Wert entspricht der Dosis, die die durch eine Gabe von 30 (mg/kg; s.c.) Isoprenalin bedingte myocardiale Radio-Calciumaufnahme zu 50 % hemmt.

Hier erwiesen sich die untersuchten neuen Verbindungen als wirksam.

Testergebnisse für folgende Verbindungen werden angegeben:

Verbindung A

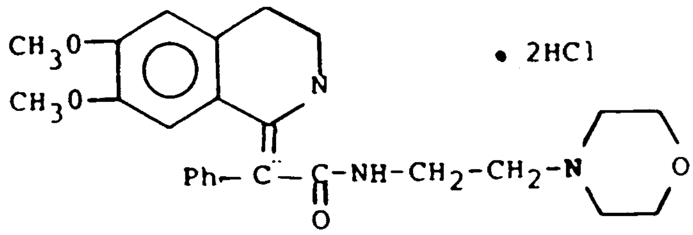

Verbindung B

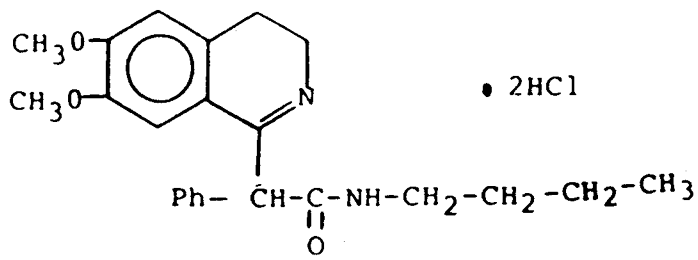

| Verbindung (perorale Verabreichung) | $H_{50}$-Wert |
|---|---|
| A | 0.73 |
| B | 0.50 |

Die Untersuchungen am isolierten Langendorff-Herzen wurden nach O. Langendorff, Pflügers Arch. 61, 291 (1895) mit kleinen Abwandlungen ausgeführt:
Isoliertes Rattenherz, retrograde Perfusion über die Aorta. Elektrische Stimulation (300 Impulse/min, Impulsdauer 1msec). Messung des linksventrikulär entwickelten Druckes mit flüssigkeitsgefülltem Latex-Ballon. Nach 20 min Aequilibrierung Auslösen eines Sauerstoff-Mangelzustandes mit 100-fach vermindertem Fluß durch Drosselung des Perfusionsflusses für 60 min, Rückkehr zu normalem Fluß bewirkt starken Anstieg der Grundspannung, unter Kontrollbedingungen setzt die Herztätigkeit nach 6 - 7 min unregelmäßig wieder ein, nach 15 - 18 min rascher Übergang zu regelmäßigen Kontraktionen. Cardioprotektiv wirksame Verbindungen verkürzen die Zeit bis zum Einsetzen regelmäßiger Kontraktionen. Infusion der Testsubstanz (gelöst in unbegastem Perfusionsmedium) während des $O_2$-Mangelzustandes herznah in das Perfusionssystem.

Die getesteten Verbindungen ergaben bei Anwendung in der unten angegebenen Konzentration eine signifikante Reduktion der Zeit bis zum Einsetzen regelmäßiger Kontraktionen.

| Verbindung | Konzentration |
|---|---|
| B (siehe oben) | 13,3 ug/ml |

In vitro-Untersuchungen an der glatten Muskulatur (Aortenstreifen; C. van Breemen, P. Aarenson, R. Lautzen heiser, K. Meisheri, Chest 78: 157S - 165S (1980); R. Casteels und G. Droogman, J. Physio. 317: 263 - 279 (1981)) haben ergeben, daß es sich bei den erfindungsgemäßen Substanzen um Calciumantagonisten mit einem neuen Wirkungsmechanismus handelt.

Aufgrund dieser Befunde sind Verbindungen der allgemeinen Formel I bzw. II und deren Salze mit Säuren, Basen ode Komplexbildnern wertvolle Wirkstoffe in Arzneimitteln mit cadioprotekiver Wirkung und können unter anderem zur Behandlung von Herzinfakten, und bei ischämischen Herzkrankheiten in der Prophylaxe und in der Therapie Anwendung finden.

Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen der Formel Ib, Ic und Id sowie deren tautomere Formen IIb', IIb'', IIc', IIc'' IId' und IId'' sind neu. Zahlreiche Verbindungen der Formel Ia, sowie deren tautomere Formen Ia' und Ia' (wie etwa diejenigen, bei denen X = $OR_1$) sind ebenfalls neu.

Die Erfindung betrifft somit auch die neuen Verbindungen der Formel

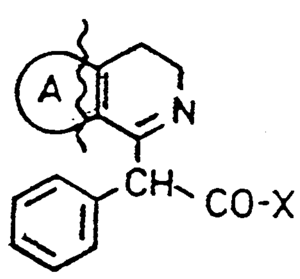

I

in der A und X wie zuvor definiert sind, mit der Maßgabe, daß Verbindungen der Formel Ia nur umfaßt sind, wenn X $OR_1$ ist.

Die Verbindungen der Formel I bzw. II können hergestellt werden

a) analog zu dem aus der EP-A 00 37 934 bekannten Verfahren durch Cyclisierung eines Phenylmalonsäurediamids der allgemeinen Formel III in der A und X wie zuvor definiert ist

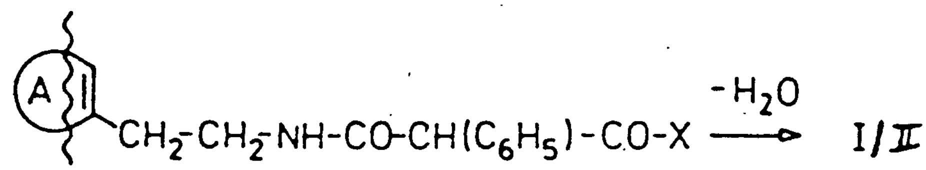


III

in Gegenwart eines Kondensationsmittels

oder

b) ausgehend von Carbonsäuren oder Carbonsäurehalogeniden der Formel IV, in der A wie zuvor definiert ist und Y für OH oder Halogen steht, durch Umsetzung mit Alkoholen bzw. Aminen der allgemeinen Formel VI, in der X wie zuvor definiert ist

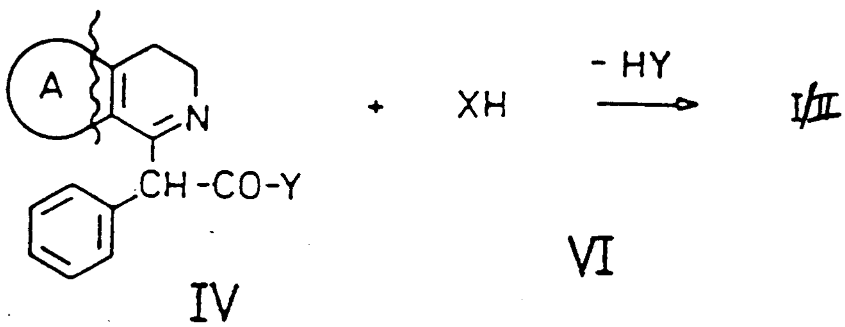


IV VI

in Gegenwart eines geeigneten Kondensationsmittels

Als Kondensationsmittel für das erfindungsgemäße Verfahren a) eignen sich zahlreiche Lewissäuren, wie z.B. Phosphoroxichlorid, Bortrifluorid, Zinntetrachlorid oder Titantetrachlorid, aber auch starke Mineralsäuren wie Schwefelsäure, Fluorsulfonsäuren, Fluorwasserstoffsäure oder Polyphosphorsäure. Sie werden gewöhnlich im Überschuß eingesetzt. Bevorzugt wird Phosphoroxichlorid.

Die Cyclisierungsreaktion kann in Gegenwart oder in Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen. Beispiele sind Benzol, Alkylbenzole, Chlorbenzole, Dekalin, Chloroform, Methylenchlorid, Acetonitril und dergl. Eine Variante des Verfahrens besteht darin, das Kondensationsmittel, beispielsweise Phosphoroxichlorid, selbst als Lösungsmittel zu verwenden.

Bezüglich der Reaktionstemperatur bestehen keine speziellen Bedingungen. Die erfindungsgemäße Umsetzung kann innerhalb eines großen Temperaturbereiches, vorzugsweise unter Erwärmen oder Erhitzen bis etwa zum Siedepunkt des Lösungsmittels durchgeführt werden.

Die Veresterungs- bzw. Amidierungsreaktion b) kann prinzipiell unter den gleichen Bedingungen wie Reaktion a) durchgeführt werden. Als Kondensationsmittel sind zusätzlich Carbodiimide - wie Cyclohexylcarbodiimid - oder Carbonyldiimidazol zu nennen.

Die als Ausgangsverbindungen für beide Verfahren dienende Phenylmalonsäuren und die Dihydropyridinylessigsäuren der Formel IV sind ebenfalls neue Verbindungen und stellen wertvolle Zwischenstufen für die Synthese der Endprodukte I bzw. II dar.

Die Erfindung betrifft somit auch Phenylmalonsäuren der Formel

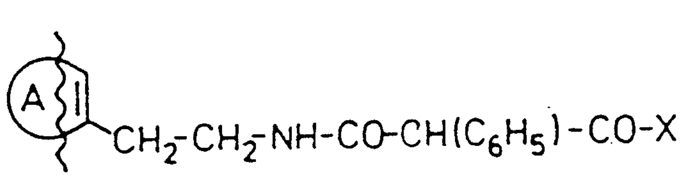

III

in der A und X wie zuvor für die neuen Verbindungen definiert sind.

Des weiteren betrifft die Erfindung Dihydropyridinylessigsäuren der Formel

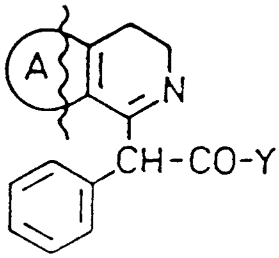

IV

sowie die dazu tautomeren Formen

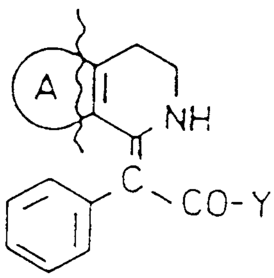

V'

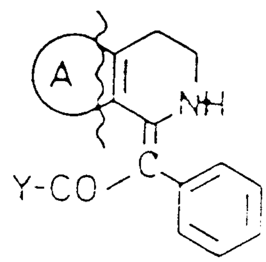

V''

V

in der Y für OH oder Halogen, vorzugsweise für Fluor, Chlor oder Brom steht und A wie zuvor definiert ist.

Die Phenylmalonsäuren der Formel III können analog zu den aus der EP-A 00 37 934 bekannten Verfahren hergestellt werden.

Dihydropyridinylessigsäuren der Formel IV bzw. V können analog zu dem allgemeinen Verfahren a) durch Cyclisierung entsprechend substituierter Phenylmalonsäuren hergestellt werden.

Beispiel 1

1-[Benzyl-alpha-(4-methyl-piperazin-1-yl-carbonyl]-3,4-dihydro-5,6,7-trimethoxy-isochinolin-Hydrochlorid.

37,8 g alpha-(4-Methylpiperazin-1-yl-carbonyl)-phenylessigsäure-2-[(3,4,5-trimethoxyphenyl)ethyl]amid werden in 120 ml Acetonitril gelöst und mit 18 ml Phosphoroxychlorid versetzt. Das Reaktionsgemisch wird ca. 2 Stunden auf Rückflußtemperatur erhitzt. Danach wird eingeengt, der Rückstand in 200 ml Methylenchlorid aufgenommen und durch Einrühren in eine Eiswasser-Pottasche-Lösung alkalisch gestellt. Nach der üblichen Aufarbeitung - ausschütteln mit Methylenchlorid, trocknen der organischen Phase über $Na_2SO_4$,abziehen des Lösungsmittels etc. wird über eine Kieselgelsäule ($CH_2Cl_2$/MeOH = 100/1 bis 100/2 gereinigt. Man isoliert die freie Base der Titelverbindung.

Zur Darstellung des Hydrochlorids wird die Base in möglichst wenig Äthanol gelöst und mit äthanolischer Salzsäure versetzt. Das Hydrochlorid wird durch tropfenweisen Zusatz von abs. Äther und Petroläther 40 : 80° (1:1) zur Kristallisation gebracht.

Man isoliert 16,5 g der Titelverbindung als Kristalle vom Schmp. 233°.

Analog darstellbar sind die nachstehend genannten Verbindungen:

Tabelle 1

Strukturtyp:

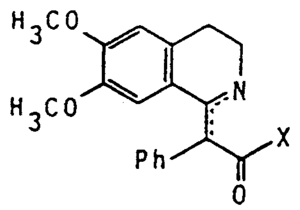

| Nr. | X | Struktur | Salzform | Fp ($^{o}C$) |
|---|---|---|---|---|
| 1. | $OCH_3$ | II | Base | 86 - 89 |
| 2. | $OC_2H_5$ | II | Base | 177 |

Tabelle 2

Strukturtyp:

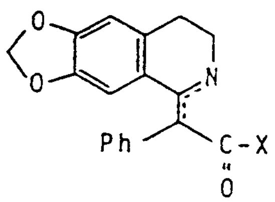

| Nr. | X | Struktur | Salzform | Fp ($^{o}C$) |
|---|---|---|---|---|
| 45. | $OC_2H_5$ | II | Base | 104 - 106 |

Tabelle 3

Strukturtyp:

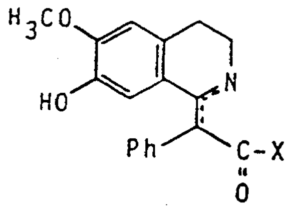

| Nr. | X | Struktur | Salzform | Fp ($^{o}C$) |
|---|---|---|---|---|
| 51. | $OC_2H_5$ | I | Base | 212 - 214 |
| | | II | Base | 208 - 211 |

Tabelle 7

Strukturtyp:

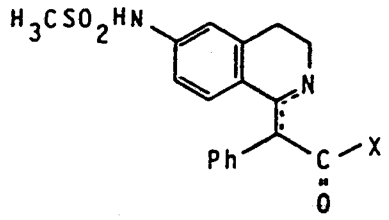

| Nr. | X | Struktur | Salzform | Fp ($^{o}C$) |
|---|---|---|---|---|
| 80 | $OC_2H_5$ | II | Base | 198 |

Tabelle 8

Strukturtyp:

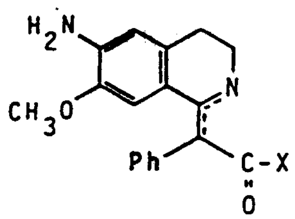

| Nr. | X | Struktur | Salzform | Fp ($^{o}C$) |
|---|---|---|---|---|
| 81. | $OC_2H_5$ | II | HCl | 183 |

Tabelle 9

Strukturtyp:

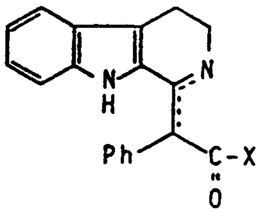

| Nr. | X | Struktur | Salzform | Fp ($^{o}C$) |
|---|---|---|---|---|
| 82. | $OC_2H_5$ | I<br>II | HCl<br>BS | 280<br>159 - 160 |
| 83. | $NH-(CH_2)_2-$ 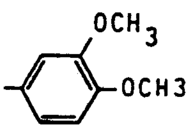 | II | BS | 64 - 70 |
| 84. | $NH-(CH_2)_2-$ 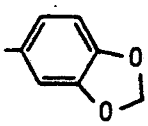 | II | BS | 156 - 160 |
| 85. | $NH-(CH_2)_2-$ 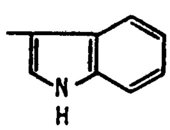 | I<br>II | BS<br>BS | 260 - 263<br>186 - 188 |
| 86. | 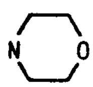 | I | BS | 133 - 135 |
| 87. | $NH-CH_2-CH(CH_3)_2$ | II | BS | 93 - 102 |
| 88. | $NH-(CH_2)_3-N(CH_3)_2$ | II | BS | 204 |
| 89. | $NH-(CH_2)_2-$ 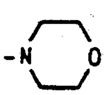 | II | BS | 140 - 143 |

Tabelle 10

Strukturtyp:

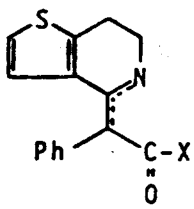

| Nr. | X | Struktur | Salzform | Fp ($^{o}C$) |
|---|---|---|---|---|
| 90. | $OC_2H_5$ | | | |
| 91. | NH-$(CH_2)_2$- 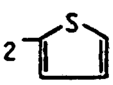 | II | BS | 1042 |

Tabelle 11

Strukturtyp:

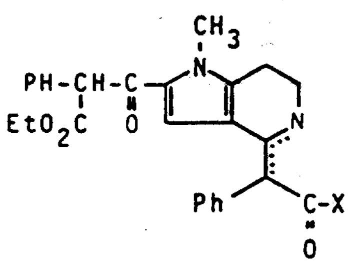

| Nr. | X | Struktur | Salzform | Fp ($^{o}C$) |
|---|---|---|---|---|
| 92. | $OC_2H_5$ | II | BS | 155 - 156 |

## Patentansprüche

1. Verwendung von carbocyclisch und heterocyclisch anellierten Dihydropyridinen der Formel

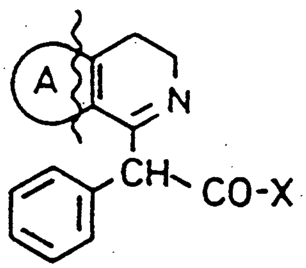

I

sowie deren tautomere Formen der Formel

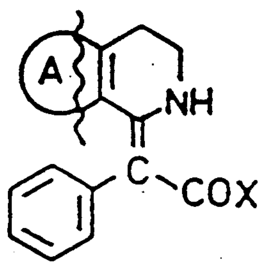

oder

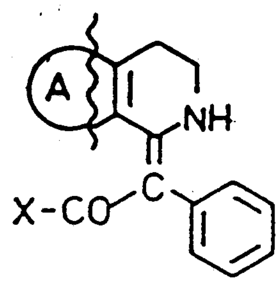

II'     II"

II

in der X für $OR_1$; $NHR_2$; $NR_3R_4$
und

| | |
|---|---|
| $R_1$ für | Wasserstoff, $C_1$-$C_4$-Alkyl; Alkoxyalkyl mit bis 4 C-Atomen |
| $R_2$ für | Wasserstoff, $C_3$-$C_6$-Alkenyl; $C_3$-$C_6$-Alkinyl; $C_3$-$C_6$-Cyclcalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c) die gleich oder verschieden sein können, substituiert ist:<br>a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können)<br>b) Phenyl; gewünschtenfalls ein- oder mehrfach gleich oder verschieden substituiert durch die Gruppen Halogen, $C_1$-$C_4$-Alkoxy, Hydroxy, Mercapto, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe -O-$(CH_2)_n$-O (mit n = 1 oder 2)<br>c) ein 5- oder 6-gliedriger gesättigter oder ganz oder teilweise ungesättigter monocyclischer Heterocyclus mit bis zu 3 Heteroatomen aus der Gruppe N, O, S; sowie als bicylischer Heterocyclus Indol (wobei die zuvor genannen Heterocyclen ein- oder mehrfach durch $C_1$-$C_4$-Alkyl substituiert sein können, $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann). |
| $R_3$ und $R_4$ | unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann. |

oder

$R_3$ und $R_4$ zusammen mit dem Stickstoffatom, an welches sie gebunden sind,für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1) substituiert sein kann;

und

$R_5$ für ein Phenylradikal, welches gewünschtenfalls wie vorstehend unter b) substituiert ist, steht;

A steht für die anellierten Ringsysteme

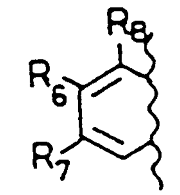
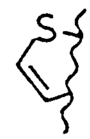
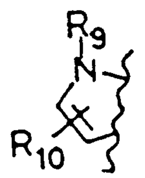
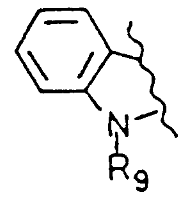

in denen

$R_8$ für Wasserstoff; $C_1$-$C_4$-Alkyl; oder $C_1$-$C_4$-Alkoxy

$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für -O-$(CH_2)_n$-O- (mit n = 1,2)

$R_9$ für Wasserstoff; $C_1$-$C_4$-Alkyl

$R_{10}$ für Wasserstoff; oder 2-Phenyl-2-ethoxycarbonylacetyl;

steht

und von Verbindungen der allgemeinen Formel I bzw. II in der

X für $NHR_2$

$R_2$ für 3-Chlorphenyl oder 2-Methyl-3-chlorphenyl;

und

A für die anellierten Ringsysteme

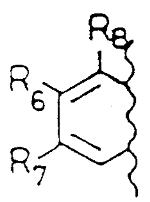
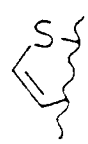
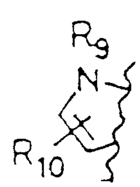
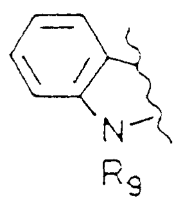

in denen

$R_8$ für Wasserstoff; oder Methoxy

$R_6$ für Methoxy; Hydroxy; Amino; oder Methansulfonylamino

$R_7$ für Wasserstoff; Methoxy; oder Hydroxy
$R_9$ für Methyl
$R_{10}$ für 2-Phenyl-2-ethxoycarbonylacetyl
steht; sowie deren Salze mit physiologisch verträglichen Säuren, Basen oder Komplexbildnern zur Herstellung von Mitteln zur Cardioprotektion.

**2.** Verwendung von carbocylisch und heterocyclisch anellierten Dihydropyridinen der Formel I bzw. II nach Anspruch 1,
in der X für $OR_1$ $NHR_2$; $NR_3R_4$
$R_1$ für Methyl oder Ethyl
$R_2$ für Wasserstoff; geradkettiges oder verzweigtes unsubstituiertes $C_1$-$C_5$-Alkyl; Allyl; Propargyl; $C_3$-$C_6$-Cycloalkyl; 3-Chlorphenyl; 2-Methyl-3-chlorphenyl; oder $C_1$-$C_3$-Alkyl, welches einfach mit einem der in nachstehend genannten Substituenten der Gruppen d) bis f) substituiert ist;
d) Cyano, Hydroxy, Methoxy, Dimethylamino
e) Phenyl, 3,4-Methylendioxyphenyl, 2,4-Dimethoxyphenyl, 4-Methoxyphenyl, 3,4-Dimethoxyphenyl, 2-Methoxyphenyl, 3-Methoxy-4-hydroxyphenyl, 3,4,5-Trimethoxyphenyl, 3-Hydroxy-4-methoxyphenyl,
f) Morpholino, Pyridin-2-yl, Indol-3-yl, Furan-2-yl, Thiophen-2-yl, Pyridin-3-yl, Pyridin-4-yl
$R_3$ und $R_4$ unabhängig voneinander für Methyl; Ethyl; 3-Cyanopropyl; Benzyl; oder 3,4,5-Trimethoxyphenethyl stehen oder
$R_3$ und $R_4$ zusammen mit dem Stickstoffatom an welches sie gebunden sind für Morpholin; Thiomorpholin; Pyrrolidin; Piperazin; 4-Methylpiperazin; 4-Benzylpiperazin; oder 4-(2-Methoxyphenyl)piperazin stehen;
und
A für die anellierten Ringsysteme

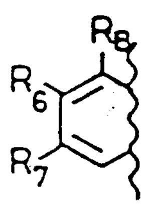

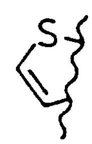

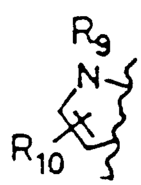

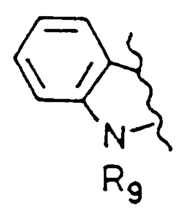

in denen
$R_8$ für Wasserstoff; oder Methoxy
$R_6$ für Methoxy; Hydroxy; Amino; oder Methansulfonylamino
$R_7$ für Wasserstoff; Methoxy; oder Hydroxy
$R_9$ für Methyl
$R_{10}$ für 2-Phenyl-2-ethoxycarbonylacetyl
steht.

**3.** Verwendung einer Verbindung der Formel I bzw. II nach Anspruch 1 oder 2, worin
A der Rest

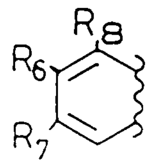

ist.

**4.** Verwendung einer Verbindung der Formel I bzw. II nach einem der Ansprüche 1 bis 3, worin $R_8$ Wasserstoff ist und $R_6$ und $R_7$ unabhängig voneinander Hydroxy oder Methoxy oder gemeinsam -O-$CH_2$-O- sind.

**5.** Verwendung einer Verbindung nach einem der Ansprüche 1 oder 2, worin X -$NHR_2$ oder -$NR_3R_4$ ist.

**6.** Verwendung einer Verbindung nach Anspruch 5, worin X-$NHR_2$ ist, worin $R_2$ $C_1$-$C_6$-alkyl ist.

**7.** Verwendung einer Verbindung nach Anspruch 6, worin $R_2$ $C_1$-$C_4$-Alkyl ist, das unsubstituiert ist oder durch Hydroxy, Phenyl (das durch Hydroxy, Methoxy oder -O-$CH_2$-O- substituiert sein kann) oder Morpholino substituiert ist.

**8.** Dihydroisochinoline der Formel

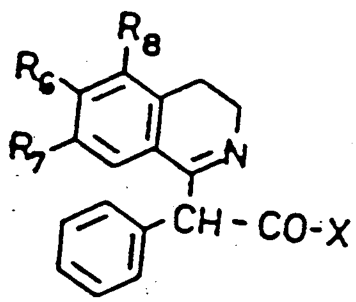

Ia

Dihydro-thieno[3,2-c]pyridine der Formel

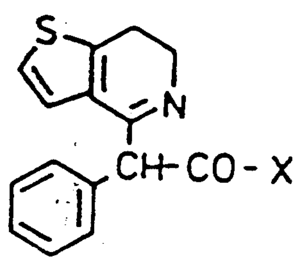

Ib

Dihydro-pyrollo[3,2-c]pyridine der Formel

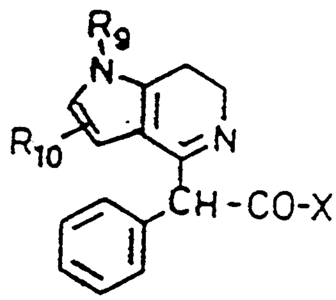

Ic

und Dihydro-pyrido[3,4-b]indole der Formel

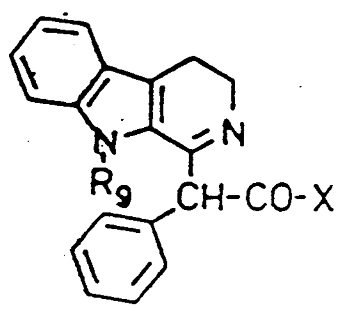

Id

sowie deren tautomere Formen die dazu E/Z-isomeren Formen und deren enatiomere Formen in der X für $OR_1$; $NHR_2$; $NR_3R_4$ , mit der Maßgabe, daß in den Verbindungen der Formel Ia X nur für $OR_1$ steht,

und

$R_1$ für — Wasserstoff, $C_1$-$C_4$-Alkyl; $C_1$-$C_4$-Alkoxyalkyl;

$R_2$ für — Wasserstoff $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c) die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können)

b) Phenyl; gewünschtenfalls ein- oder mehrfach gleich oder verschieden substituiert durch die Gruppen Halogen, $C_1$-$C_4$-Alkoxy, Hydroxy, Mercapto, $C_1$-$C_4$-Alkylthio,

$C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe -O-$(CH_2)_n$-O(mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S; sowie als bicylischer Heterocyclus Indol (wobei die zuvor genannen Heterocyclen ein- oder mehrfach durch $C_1$-$C_4$-Alkyl; $C_3$-$C_6$-Cycloalkyl; $C_5$- oder $C_6$-Cycloalkenyl; $C_2$-$C_3$-Acyl;

$C_1$-$C_4$-Alkylsulfonyl; oder Phenyl (welches seinerseits bis zu dreifach wie unter b) beschrieben substituiert sein kann) substituiert sein können;

$R_3$ und $R_4$ — unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ — zusammen mit dem Stickstoffatom, an welches sie gebunden sind für einen ganz- oder teilweise gesättigten heterocyclischen 5- oder 6-Ring (der außerdem noch bis zu

2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0,1) substituiert sein kann;

und

- $R_5$ für ein Phenylradikal, welches gewünschtenfalls wie vorstehend unter b) substituiert ist, steht;
- $R_8$ für Wasserstoff; $C_1$-$C_4$-Alkyl; oder $C_1$-$C_4$-Alkoxy
- $R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

- $R_6$ und $R_7$ gemeinsam für -O-$(CH_2)_n$-O- (mit n = 1 oder 2)
- $R_9$ für Wasserstoff, $C_1$-$C_4$-Alkyl
- $R_{10}$ für Wasserstoff; oder 2-Phenyl-2-ethoxycarbonylacetyl;

**9.** Verfahren zur Herstellung von Verbindungen der Formel Ia bis Id und deren tautomere Formen nach Anspruch 8 a) durch Cyclisierung eines Phenylmalonsäureamids der allgemeinen Formel III

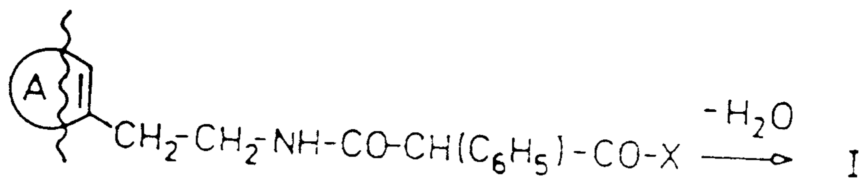


III

in Gegenwart einer Lewissäure als Kondensationsmittel

oder

b) ausgehend von Carbonsäuren oder Carbonsäurehalogeniden der Formel IV, in der A wie zuvor definiert ist und Y für OH oder Halogen steht, durch Umsetzung mit Alkohlen bzw. Aminen der Allgemeinen Formel VI, in der X wie zuvor definiert ist.

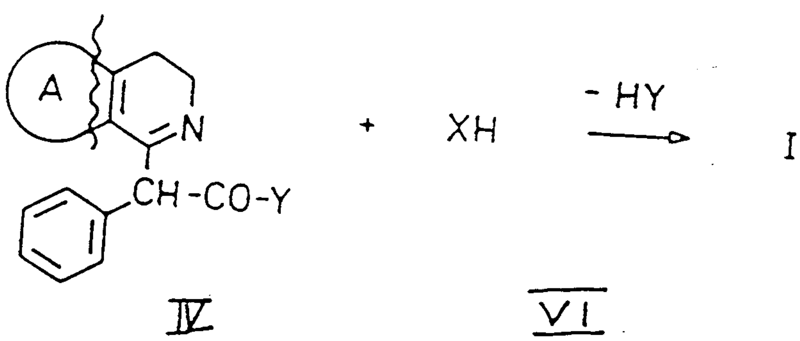


IV VI

in Gegenwart eines geeigneten Carbodiimides oder Carbonyldiimidazols als Kondensationsmittel.

**10.** Dihydropyridinylessigsäuren der Formel

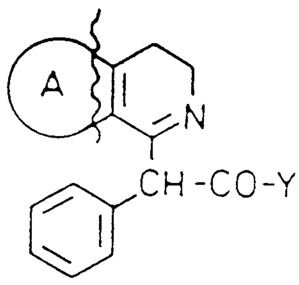

IV

sowie die dazu tautomeren Formen

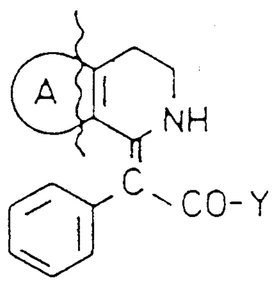

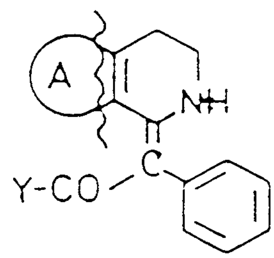

V' V'

V.

in der Y für OH oder Halogen
und
A für die anellierten Ringsysteme

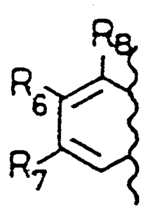

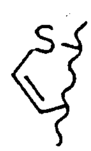

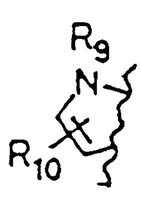

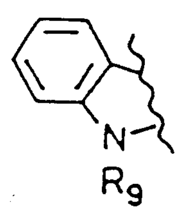

in denen

$R_8$ für Wasserstoff; $C_1$-$C_4$-Alkyl; oder $C_1$-$C_4$-Alkoxy

$R_6$ und $R_7$ die gleich oder verschieden sein können für Wasserstoff; Hydroxy; $C_1$-$C_4$-Alkyl; $C_1$-$C_4$-Alkoxy; Amino; Methansulfonylamino

oder

$R_6$ und $R_7$ gemeinsam für $-O-(CH_2)_n-O-$ (mit n = 1,2)

$R_9$ für Wasserstoff; $C_1$-$C_4$-Alkyl

$R_{10}$ für Wasserstoff; oder 2-Phenyl-2-ethoxycarbonylacetyl

steht, sowie deren Salze mit Säuren und Basen.

**11.** Verbindung der allgemeinen Formel IV bzw. V nach Anspruch 10, worin Y Fluor, Chlor oder Brom ist.

**12.** Phenylmalonsäuren der Formel III,

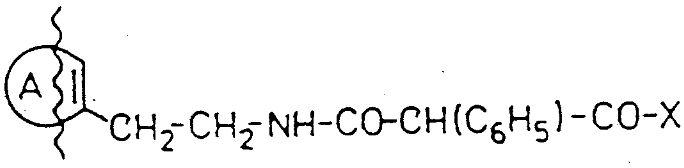

III

in der X für $OR_1$; $NHR_2$; $NR_3R_4$

und

$R_1$ für Wasserstoff, $C_1$-$C_4$-Alkyl; Alkoxyalkyl mit bis 4 C-Atomen;

$R_2$ für Wasserstoff $C_3$-$C_6$-Alkenyl; $C_3$-$C_6$-Alkinyl; $C_3$-$C_6$-Cycloalkyl; $C_3$-$C_6$-Cycloalkenyl; geradkettiges oder verzweigtes $C_1$-$C_6$-Alkyl, welches gewünschtenfalls ein- oder mehrfach mit den nachstehend genannten Substituenten der Gruppen a) bis c) die gleich oder verschieden sein können, substituiert ist:

a) Halogen; Cyano; Hydroxy; Mercapto; $C_1$-$C_4$-Alkoxy; $C_1$-$C_4$-Alkylthio; Amino; Mono-$C_1$-$C_4$-Alkylamino; Di-$C_1$-$C_4$-Alkylamino (wobei die Alkylradikale gleich oder verschieden sein können)

b) Phenyl; gewünschtenfalls ein- oder mehrfach gleich oder verschieden substituiert durch die Gruppen Halogen, $C_1$-$C_4$-Alkoxy, Hydroxy, Mercapto, $C_1$-$C_4$-Alkylthio, $C_1$-$C_4$-Alkylthio, $C_1$-$C_4$-Alkyl, Amino, Mono-$C_1$-$C_4$-Alkylamino, Di-$C_1$-$C_4$-Alkylamino (wobei die Alkylreste gleich oder verschieden sein können), $C_2$-$C_3$-Acylamino, $C_2$-$C_3$-Acyloxy sowie die vicinal an das Phenylsystem gebundene Gruppe $-O-(CH_2)_n-O$(mit n = 1 oder 2)

c) ein 5- oder 6-gliedriger gesättigter oder ganz oder teilweise ungesättigter monocyclischer Heterocyclus mit bis zu 3 Heteroatomen aus der Gruppe N, O, S sowie als bicyclischer Heterocyclus Indol (wobei die zuvor genannen Heterocyclen ein- oder mehrfach durch $C_1$-$C_4$-Alkyl substituiert sein können $C_3$-$C_6$-Cycloalkyl; $C_5$- oder $C_6$-Cycloalkenyl; $C_2$-$C_3$-Acyl; $C_1$-$C_4$-Alkylsulfonyl; oder Phenyl (welches seinerseits bis zu dreifach wie unter b) beschrieben substituiert sein kann);

$R_3$ und $R_4$ unabhängig voneinander für $C_1$-$C_4$-Alkyl, welches gewünschtenfalls phenylsubstituiert sein kann, wobei der Phenylsubstituent seinerseits wie vorstehend unter b) substituiert sein kann;

oder

$R_3$ und $R_4$ zusammen mit dem Stickstoffatom, an welches sie gebunden sind für einen ganz- oder teilweise gesättigten heterocyclischen 5- oder 6-Ring (der außerdem noch bis zu 2 weitere Heteroatome aus der Gruppe N, O, S enthalten kann) bilden, wobei der so erhaltene Heterocyclus durch $C_1$-$C_4$-Alkyl, Hydroxy, oder $(CH_2)_p$-$R_5$ (mit p = 0 oder 1), substituiert sein kann;

und

$R_5$ für ein Phenylradikal, welches gewünschtenfalls wie vorstehend unter b) substituiert ist, steht;

A steht für die anellierten Ringsysteme

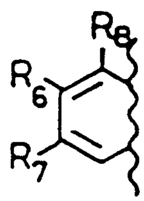

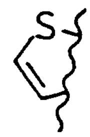

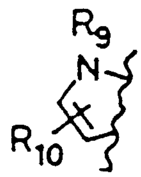

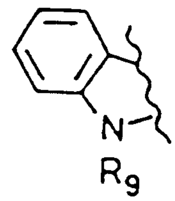

in denen

| | |
|---|---|
| $R_8$ | für Wasserstoff; $C_1$-$C_4$-Alkyl; oder $C_1$-$C_4$-Alkoxy |
| $R_6$ und $R_7$ | die gleich oder verschieden sein können für Wasserstoff; Hydroxy; $C_1$-$C_4$-Alkyl; $C_1$-$C_4$-Alkoxy; Amino; Methansulfonylamino |

oder

| | |
|---|---|
| $R_6$ und $R_7$ | gemeinsam für -O-$(CH_2)_n$-O- (mit n = 1,2) |
| $R_9$ | für Wasserstoff; $C_1$-$C_4$-Alkyl |
| $R_{10}$ | für Wasserstoff; oder 2-Phenyl-2-ethoxycarbonylacetyl; |

steht,
mit der Maßgabe, daß in den Verbindungen, worin
A für das Ringsystem

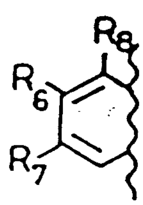

steht, X nur für $OR_1$ steht.

## Claims

**1.** Use of carbocyclically and heterocyclically anellated dihydropyridines of formula

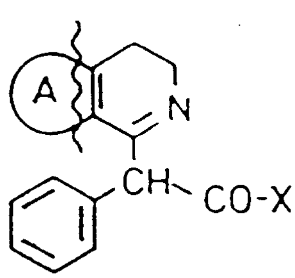

I

and the tautomeric forms thereof of formula

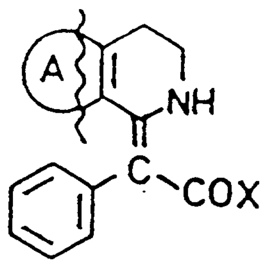

or

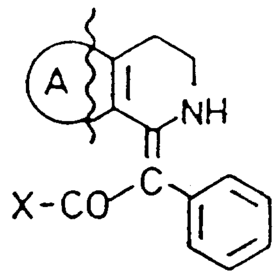

II' II''

II

wherein X represents $OR_1$; $NHR_2$; $NR_3R_4$

and

$R_1$ represents hydrogen, $C_{1-4}$ alkyl; alkoxyalkyl having up to 4 carbon atoms;

$R_2$ represents hydrogen; $C_{3-6}$ alkenyl; $C_{3-6}$ alkynyl; $C_{3-6}$ cycloalkyl; $C_{3-6}$ cycloalkenyl; straight-chained or branched $C_{1-6}$ alkyl which may, if desired, be mono- or polysubstituted by the following substituents of groups a) to c), which may be identical or different:

a) halogen; cyano; hydroxy; mercapto; $C_{1-4}$ alkoxy; $C_{1-4}$ alkylthio; amino; mono-$C_{1-4}$ alkylamino; di-$C_{1-4}$ alkylamino (whilst the alkyl radicals may be identical or different;

b) phenyl; optionally mono- or polysubstituted, by either identical or different substituents, by the groups halogen, $C_{1-4}$ alkoxy, hydroxy, mercapto, $C_{1-4}$ alkylthio, $C_{1-4}$ alkyl, amino, mono-$C_{1-4}$ alkylamino, di-$C_{1-4}$ alkylamino (wherein the alkyl groups may be identical or different), $C_{2-3}$ acylamino, $C_{2-3}$ acyloxy and the group $-O-(CH_2)_n-O-$ (wherein n = 1 or 2) which is vicinally bonded to the phenyl system

c) a 5- or 6-membered saturated or wholly or partially unsaturated monocyclic heterocyclic group with up to 3 heteroatoms selected from the group comprising N, O, S; and, as a bicyclic heterocyclic group, indole (whilst the above-mentioned heterocyclic groups may be mono- or polysubstituted by $C_{1-4}$ alkyl; $C_{3-6}$ cycloalkyl; $C_5$ or $C_6$ cycloalkenyl; $C_{2-3}$ acyl; $C_{1-4}$ alkylsulphonyl; or phenyl (which may in turn be substituted up to three times as described in b)).

$R_3$ and $R_4$ independently of each other represent $C_{1-4}$ alkyl, which may, if desired, be phenyl-substituted, whilst the phenyl substituent may in turn be substituted as in b) hereinbefore.

or

$R_3$ and $R_4$ together with the nitrogen atom to which they are bonded may represent a wholly or partially saturated heterocyclic 5- or 6-membered ring (which may also contain up to 2 further heteroatoms selected from the group comprising N, O, S), whilst the resulting heterocyclic group may be substituted by $C_{1-4}$ alkyl, hydroxy or $(CH_2)_p-R_5$ - (wherein p = 0 or 1)

and

$R_5$ represents a phenyl radical which is optionally substituted as in b) hereinbefore;

A represents the anellated ring systems

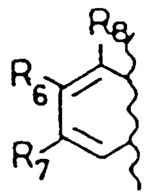
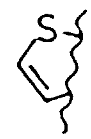
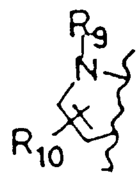
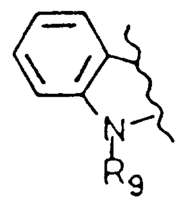

wherein

- $R_8$ represents hydrogen; $C_{1-4}$ alkyl; or $C_{1-4}$ alkoxy
- $R_6$ and $R_7$ which may be identical or different represent hydrogen, hydroxy, $C_{1-4}$ alkyl, $C_{1-4}$ alkoxy, amino, methanesulphonylamino

or

- $R_6$ and $R_7$ together represent $-O-(CH_2)_n-O-$ (wherein n = 1 or 2)
- $R_9$ represents hydrogen, $C_{1-4}$ alkyl
- $R_{10}$ represents hydrogen or 2-phenyl-2-ethoxycarbonylacetyl

and compounds of general formula I or II wherein

- X represents $NHR_2$,
- $R_2$ represents 3-chlorophenyl or 2-methyl-3-chlorophenyl;

and

- A represents the anellated ring systems

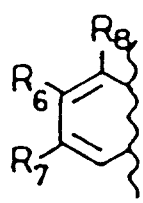
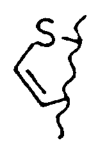
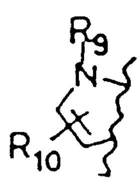
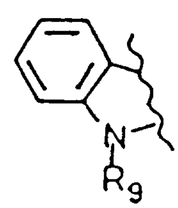

wherein

- $R_8$ represents hydrogen or methoxy;
- $R_6$ represents methoxy, hydroxy, amino or methanesulphonylamino;
- $R_7$ represents hydrogen, methoxy or hydroxy;
- $R_9$ represents methyl;
- $R_{10}$ represents 2-phenyl-2-ethoxycarbonylacetyl;

and the salts thereof with physiologically acceptable acids, bases or complexing agents for preparing agents for cardioprotection.

**2.** Use of carbocyclically and heterocyclically anellated dihydropyridines of formula I or II as claimed in claim 1
wherein X represents $OR_1$ $NHR_2$; $NR_3R_4$

$R_1$ represents methyl or ethyl

$R_2$ represents hydrogen, Straight-chained or branched unsubstituted $C_{1-5}$ alkyl; allyl; propargyl; $C_{3-6}$ cycloalkyl; 3-chlorophenyl; 2-methyl-3-chlorophenyl; or $C_{1-3}$ alkyl which is monosubstituted by one of the substituents listed below in groups d) to f);

d) cyano, hydroxy, methoxy, dimethylamino

e) phenyl, 3,4-methylenedioxyphenyl, 2,4-dimethoxyphenyl, 4-methoxyphenyl, 3,4-dimethoxyphenyl, 2-methoxyphenyl, 3-methoxy-4-hydroxyphenyl, 3,4,5-trimethoxyphenyl, 3-hydroxy-4-methoxyphenyl,

f) morpholino, pyridin-2-yl, indol-3-yl, furan-2-yl, thiophen-2-yl, pyridin-3-yl, pyridin-4-yl

$R_3$ and $R_4$ independently of each other represent methyl; ethyl; 3-cyanopropyl; benzyl, or 3,4,5-trimethoxyphenethyl or

$R_3$ and $R_4$ together with the nitrogen atom to which they are bonded represent morpholine; thiomorpholine; pyrrolidine; piperazine; 4-methylpiperazine; 4-benzylpiperazine; or 4-(2-methoxyphenyl)piperazine;

and

A represents the anellated ring systems

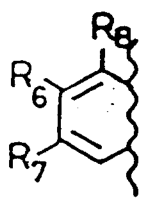

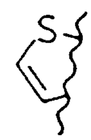

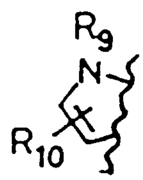

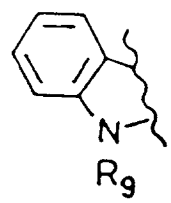

wherein

$R_8$ represents hydrogen or methoxy

$R_6$ represents methoxy, hydroxy, amino or methanesulphonylamino

$R_7$ represents hydrogen, methoxy or hydroxy

$R_9$ represents methyl

$R_{10}$ represents 2-phenyl-2-ethoxycarbonylacetyl.

**3.** Use of a compound of formula I or II as claimed in claim 1 or 2 wherein A is the group

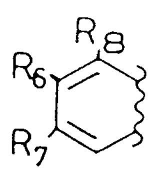

**4.** Use of a compound of formula I or II according to one of claims 1 to 3, wherein $R_8$ is hydrogen and $R_6$ and $R_7$ independently of each other represent hydroxy or methoxy or together represent $-O-CH_2-O-$.

**5.** Use of a compound as claimed in one of claims 1 or 2 wherein X is $-NHR_2$ or $-NR_3R_4$.

**6.** Use of a compound as claimed in claim 5 wherein X is $-NHR_2$, wherein $R_2$ is $C_{1-6}$ alkyl.

7. Use of a compound as claimed in claim 6 wherein $R_2$ is $C_{1-4}$ alkyl which is unsubstituted or substituted by hydroxy, phenyl (which may be substituted by hydroxy, methoxy or $-O-CH_2-O-$) or morpholino.

8. Dihydroisoquinolines of formula

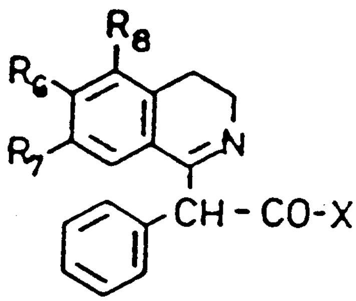

Ia

dihydro-thieno[3,2-c]pyridines of formula

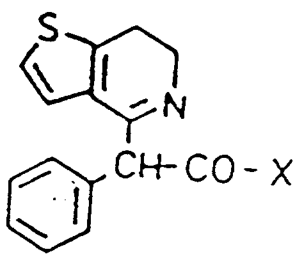

Ib

dihydropyrrolo[3,2-c]pyridines of formula

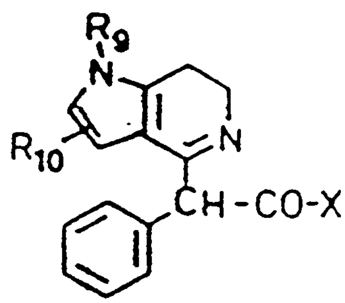

Ic

and dihydropyrido[3,4-b]indoles of formula

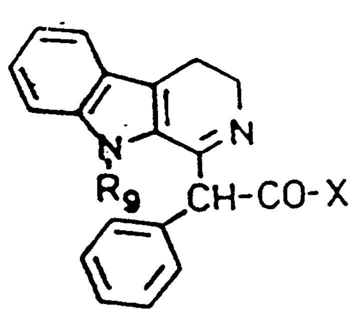

Id

and the tautomeric forms thereof, their E/Z-isomeric forms and their enantiomeric forms

wherein X represents $OR_1$, $NHR_2$; $NR_3R_4$, with the proviso that, in the compounds of formula Ia, X represents only $OR_1$, and

$R_1$ represents hydrogen, $C_{1-4}$ alkyl; $C_{1-4}$ alkoxyalkyl

$R_2$ represents hydrogen; $C_{3-6}$ alkenyl; $C_{3-6}$ alkynyl; $C_{3-6}$ cycloalkyl; $C_{3-6}$ cycloalkenyl; straight-chained or branched $C_{1-6}$ alkyl which may, if desired, be mono- or polysubstituted by the following substituents of groups a) to c), which may be identical or different:

a) halogen; cyano; hydroxy; mercapto; $C_{1-4}$ alkoxy; $C_{1-4}$ alkylthio; amino; mono-$C_{1-4}$ alkylamino; di-$C_{1-4}$ alkylamino (whilst the alkyl radicals may be identical or different;

b) phenyl; optionally mono- or polysubstituted, by either identical or different substituents, by the groups halogen, $C_{1-4}$ alkoxy, hydroxy, mercapto, $C_{1-4}$ alkylthio, $C_{1-4}$ alkyl, amino, mono-$C_{1-4}$ alkylamino, di-$C_{1-4}$ alkylamino (wherein the alkyl groups may be identical or different), $C_{2-3}$ acylamino, $C_{2-3}$ acyloxy and the group $-O-(CH_2)_n-O$ (wherein n = 1 or 2) which is vicinally bonded to the phenyl system

c) a 5- or 6-membered saturated or wholly or partially unsaturated monocyclic heterocyclic group with up to 3 heteroatoms selected from the group comprising N, O, S; and, as a bicyclic heterocyclic group, indole (whilst the above-mentioned heterocyclic groups may be mono- or polysubstituted by $C_{1-4}$ alkyl; $C_{3-6}$ cycloalkyl; $C_5$ or $C_6$ cycloalkenyl; $C_{2-3}$ acyl; $C_{1-4}$ alkylsulphonyl; or phenyl (which may in turn be substituted up to three times as described in b)).

$R_3$ and $R_4$ independently of each other represent $C_{1-4}$ alkyl, which may, if desired, be phenyl-substituted, whilst the phenyl substituent may in turn be substituted as in h) hereinbefore.

or

$R_3$ and $R_4$ together with the nitrogen atom to which they are bonded may represent a wholly or partially saturated heterocyclic 5- or 6-membered ring (which may also contain up to 2 further heteroatoms selected from the group comprising N, O, S), whilst the resulting heterocyclic group may be substituted by $C_{1-4}$ alkyl, hydroxy or $(CH_2)_p-R_5$ - (wherein p = 0 or 1)

and

$R_5$ represents a phenyl radical which is optionally substituted as in b) hereinbefore;

$R_8$ represents hydrogen; $C_{1-4}$ alkyl; or $C_{1-4}$ alkoxy

$R_6$ and $R_7$ which may be identical or different represent hydrogen, hydroxy, $C_{1-4}$ alkyl, $C_{1-4}$ alkoxy, amino, methanesulphonylamino

or

$R_6$ and $R_7$ together represent $-O-(CH_2)_n-O-$ (wherein n = 1 or 2)

$R_9$ represents hydrogen, $C_{1-4}$ alkyl

$R_{10}$ represents hydrogen or 2-phenyl-2-ethoxycarbonylacetyl.

**9.** Process for preparing compounds of formulae Ia to Id and their tautomeric forms as claimed in claim 8
a) by cyclising a phenylmalonic acid amide of general formula III

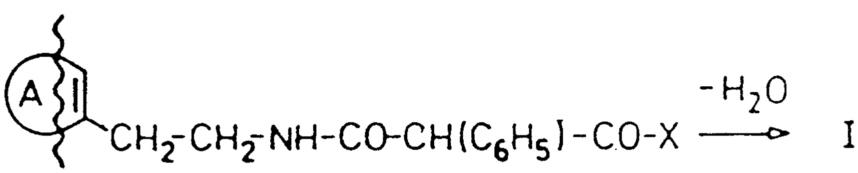

III

in the presence of a Lewis acid as condensing agent

or

b) starting from carboxylic acids or carboxylic acid halides of formula IV wherein A is defined as hereinbefore and Y represents OH or halogen, by reacting with alcohols or amines of general

formula VI wherein X is defined as hereinbefore

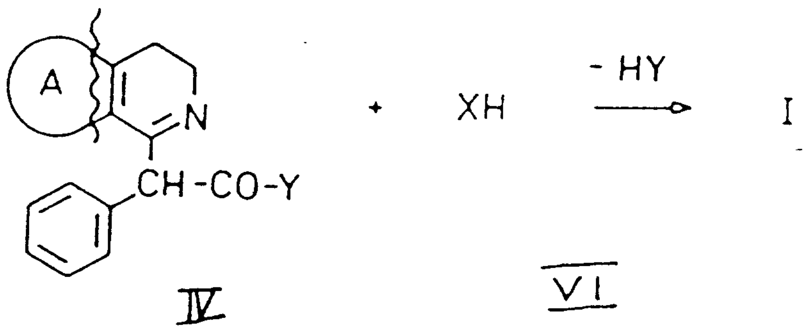


IV VI

in the presence of a suitable carbodiimide or carbonyldiimidazole as condensing agent.

**10.** Dihydropyridinyl acetic acids of formula

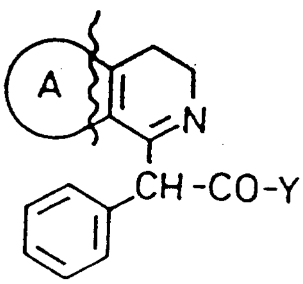

IV (IV)

and the tautomeric forms

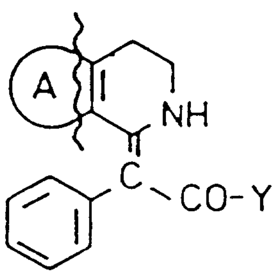

V'

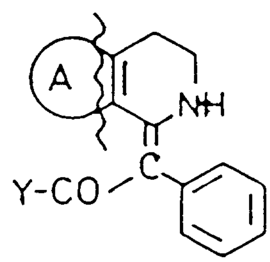

V'

V

wherein Y represents OH or halogen and
A represents the anellated ring systems

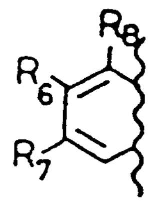

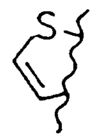

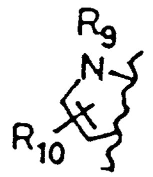

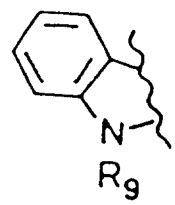

wherein

| | |
|---|---|
| $R_8$ | represents hydrogen; $C_{1-4}$ alkyl; or $C_{1-4}$ alkoxy |
| $R_6$ and $R_7$ | which may be identical or different represent hydrogen, hydroxy, $C_{1-4}$ alkyl, $C_{1-4}$ alkoxy, amino, methanesulphonylamino |

or

| | |
|---|---|
| $R_6$ and $R_7$ | together represent $-O-(CH_2)_n-O-$ (wherein n = 1,2) |
| $R_9$ | represents hydrogen, $C_{1-4}$ alkyl |
| $R_{10}$ | represents hydrogen or 2-phenyl-2-ethoxycarbonylacetyl |

and the salts thereof with acids and bases.

**11.** Compound of general formula IV or V according to claim 10 wherein Y is fluorine, chlorine or bromine.

**12.** Phenylmalonic acids of formula III

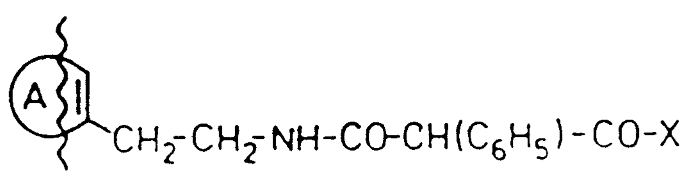

III

wherein X represents $OR_1$; $NHR_2$; $NR_3R_4$
and
$R_1$ represents hydrogen, $C_{1-4}$ alkyl; alkoxyalkyl having up to 4 carbon atoms;
$R_2$ represents hydrogen; $C_{3-6}$ alkenyl; $C_{3-6}$ alkynyl;
$C_{3-6}$ cycloalkyl; $C_{3-6}$ cycloalkenyl; straight-chained or branched $C_{1-6}$ alkyl which may, if desired, be mono- or polysubstituted by the following substituents of groups a) to c), which may be identical or different:

a) halogen; cyano; hydroxy; mercapto; $C_{1-4}$ alkoxy; $C_{1-4}$ alkylthio; amino; mono-$C_{1-4}$ alkylamino; di-$C_{1-4}$ alkylamino (whilst the alkyl radicals may be identical or different;

b) phenyl; optionally mono- or polysubstituted, by either identical or different substituents, by the groups halogen, $C_{1-4}$ alkoxy, hydroxy, mercapto, $C_{1-4}$ alkylthio, $C_{1-4}$ alkyl, amino, mono-$C_{1-4}$ alkylamino, di-$C_{1-4}$ alkylamino (wherein the alkyl groups may be identical or different), $C_{2-3}$ acylamino, $C_{2-3}$ acyloxy and the group $-O-(CH_2)_n-O$ (wherein n = 1 or 2) which is vicinally bonded to the phenyl system

c) a 5- or 6-membered saturated or wholly or partially unsaturated monocyclic heterocyclic group with up to 3 heteroatoms selected from the group comprising N, O, S; and, as a bicyclic heterocyclic group, indole (whilst the above-mentioned heterocyclic groups may be mono- or polysubstituted by $C_{1-4}$ alkyl; $C_{3-6}$ cycloalkyl; $C_5$ or $C_6$ cycloalkenyl; $C_{2-3}$ acyl; $C_{1-4}$ alkylsulphonyl; or phenyl (which may in turn be substituted up to three times as described in b)).

$R_3$ and $R_4$ independently of each other represent $C_{1-4}$ alkyl, which may, if desired, be phenyl-substituted, whilst the phenyl substituent may in turn be substituted as in b) hereinbefore;

or

$R_3$ and $R_4$ together with the nitrogen atom to which they are bonded may represent a wholly or partially saturated heterocyclic 5- or 6-membered ring (which may also contain up to 2 further heteroatoms selected from the group comprising N, O, S), whilst the resulting heterocyclic group may be substituted by $C_{1-4}$ alkyl, hydroxy or $(CH_2)_p$-$R_5$ - (wherein p = 0 or 1)

and

$R_5$ represents a phenyl radical which is optionally substituted as in b) hereinbefore;

A represents the anellated ring systems

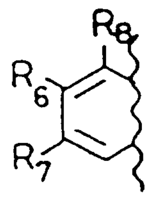
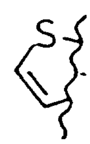
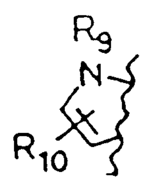
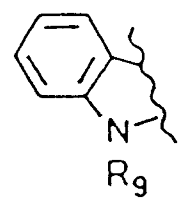

wherein

$R_8$ represents hydrogen; $C_{1-4}$ alkyl; or $C_{1-4}$ alkoxy

$R_6$ and $R_7$ which may be identical or different represent hydrogen, hydroxy, $C_{1-4}$ alkyl, $C_{1-4}$ alkoxy, amino, methanesulphonylamino

or

$R_6$ and $R_7$ together represent -O-$(CH_2)_n$-O- (wherein n = 1,2)

$R_9$ represents hydrogen, $C_{1-4}$ alkyl

$R_{10}$ represents hydrogen or 2-phenyl-2-ethoxycarbonylacetyl

with the proviso that in the compounds wherein

A represents the ring system

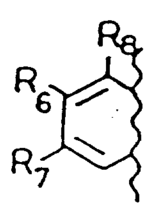

X represents only $OR_1$.

## Revendications

**1.** Utilisation de dihydropyridines à condensation carbocyclique et hétérocyclique de formule

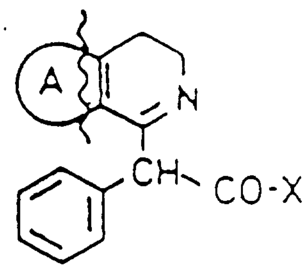

I

et de leurs formes tautomères de formule

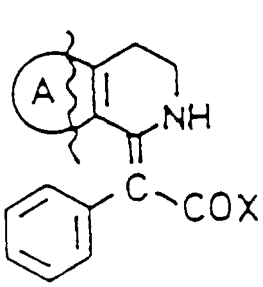

ou

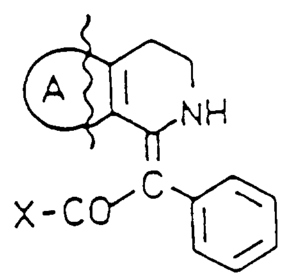

II' II''

II

où X représente $OR_1$, $NHR_2$, $NR_3R_4$
et

$R_1$ est l'hydrogène, alkyle en $C_1$-$C_4$; alcoxyalkyle ayant jusqu'à 4 atomes de carbone;

$R_2$ est l'hydrogène, alcényle en $C_3$-$C_6$; alcynyle en $C_3$-$C_6$; cycloalkyle en $C_3$-$C_6$; cycloalcényle en $C_3$-$C_6$; alkyle en $C_1$-$C_6$ linéaire ou ramifié qui est éventuellement substitué une ou plusieurs fois par les substituants des groupes a) à c), cités ci-dessous, et qui peuvent être identiques ou différents:

a) halogène; cyano; hydroxy; mercapto; alcoxy en $C_1$-$C_4$; alkylthio en $C_1$-$C_4$; amino; mono(alkyl en $C_1$-$C_4$)amino; di(alkyl en $C_1$-$C_4$)amino (où les radicaux alkyle peuvent être identiques ou différents);

b) phényle, éventuellement substitué une ou plusieurs fois de façon identique ou différente par les groupes halogène, alcoxy en $C_1$-$C_4$, hydroxy, mercapto, alkylthio en $C_1$-$C_4$, alkyle en $C_1$-$C_4$, amino, mono(alkyl en $C_1$-$C_4$)amino, di(alkyl en $C_1$-$C_4$)amino (les restes alkyle pouvant être identiques ou différents), acylamino en $C_2$-$C_3$, acyloxy en $C_2$-$C_3$, et le groupe $-O-(CH_2)_n-O-$ (avec n = 1 ou 2) lié au système du phényle en des positions adjacentes;

c) un hétérocycle monocyclique de 5 ou 6 chaînons saturé ou totalement ou partiellement insaturé, ayant jusqu'à 3 hétéroatomes du groupe N, O, S; et l'indole comme hétérocycle bicyclique (les hétérocycles précités pouvant être substitués une ou plusieurs fois par alkyle en $C_1$-$C_4$, cycloalkyle en $C_3$-$C_6$; cycloalcényle en $C_5$ ou $C_6$; acyle en $C_2$-$C_3$; alkylsulfonyle en $C_1$-$C_4$; ou phényle (qui peut à son tour être substitué jusqu'à trois fois de la manière décrite sous b));

$R_3$ et $R_4$, indépendamment l'un de l'autre, sont alkyle en $C_1$-$C_4$, qui peut éventuellement être substitué par phényle, le substituant phényle pouvant à son tour être substitué comme sous b) ci-dessus; ou bien

$R_3$ et $R_4$ forment ensemble avec l'atome d'azote auquel ils sont liés un noyau hétérocyclique de 5 ou 6 chaînons, totalement ou partiellement saturé (qui peut contenir en outre jusqu'à 2 autres

hétéroatomes du groupe N, O, S), l'hétérocycle ainsi obtenu pouvant être substitué par alkyle en $C_1$-$C_4$, hydroxy, ou $(CH_2)_p$-$R_5$ (avec p = 0 ou 1); et

$R_5$ représente un radical phényle qui est éventuellement substitué comme sous b) ci-dessus;

A représente les systèmes cycliques condensés

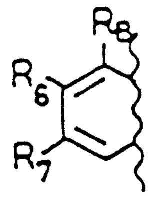

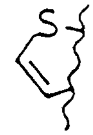

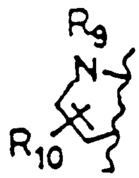

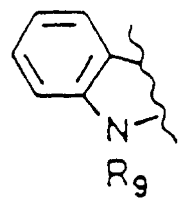

dans lesquels

$R_8$ est l'hydrogène; alkyle en $C_1$-$C_4$; ou alcoxy en $C_1$-$C_4$;

$R_6$ et $R_7$, qui peuvent être identiques ou différents, sont l'hydrogène; hydroxy; alkyle en $C_1$-$C_4$; alcoxy en $C_1$-$C_4$; amino; méthanesulfonylamino; ou bien

$R_6$ et $R_7$ forment ensemble -O-$(CH_2)_n$-O- (avec n = 1, 2)

$R_9$ est l'hydrogène; alkyle en $C_1$-$C_4$;

$R_{10}$ est l'hydrogène; ou 2-phényl-2-éthoxycarbonylacétyle;

et de composés de formule générale I ou II où

X est $NHR_2$

$R_2$ est 3-chlorophényle ou 2-méthyl-3-chlorophényle; et

A représente les systèmes cycliques condensés

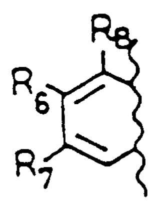

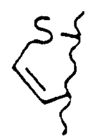

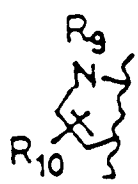

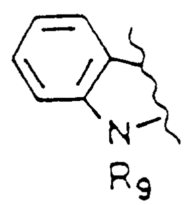

dans lesquels

$R_8$ est l'hydrogène ou méthoxy;

$R_6$ est méthoxy; hydroxy; amino; ou méthanesulfonylamino;

$R_7$ est l'hydrogène; méthoxy; ou hydroxy;

$R_9$ est méthyle;

$R_{10}$ est 2-phényl-2-éthoxycarbonylacétyle;

ainsi que de leurs sels avec des acides, bases ou agents complexants physiologiquement acceptables, pour la préparation de compositions cardioprotectrices.

**2.** Utilisation de dihydropyridines à condensation carbocyclique et hétérocyclique de formule I ou II selon la revendication 1, où

X est $OR_1$; $NHR_2$; ou $NR_3R_4$;

$R_1$ est méthyle ou éthyle;

$R_2$ est l'hydrogène, alkyle en $C_1$-$C_5$ non substitué linéaire ou ramifié; allyle; propargyle; cycloalkyle en $C_3$-$C_6$; 3-chlorophényle; 2-méthyl-3-chlorophényle; ou alkyle en $C_1$-$C_3$ qui est monosubstitué par l'un des substituants des groupes d) à f), cités ci-dessous:

d) cyano, hydroxy, méthoxy, diméthylamino

e) phényle, 3,4-méthylènedioxyphényle, 2,4-diméthoxyphényle, 4-méthoxyphényle, 3,4-diméthoxyphényle, 2-méthoxyphényle, 3-méthoxy-4-hydroxyphényle 3,4,5-triméthoxyphényle, 3-hydroxy-4-méthoxyphényle,

f) morpholino pyridine-2-yle, indole-3-yle, furane-2-yle, thiophène-2-yle, pyridine-3-yle, pyridine-4-yle

$R_3$ et $R_4$, indépendamment l'un de l'autre, sont méthyle; éthyle; 3-cyanopropyle; benzyle; ou 3,4,5-triméthoxyphénéthyle; ou bien

$R_3$ et $R_4$, ensemble avec l'atome d'azote auquel ils sont liés, représentent un cycle morpholine; thiomorpholine; pyrrolidine; pipérazine; 4-méthylpipérazine; 4-benzylpipérazine; ou 4-(2-méthoxyphényl)pipérazine; et

A représente les systèmes cycliques condensés

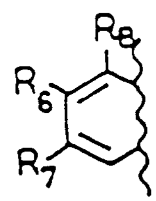

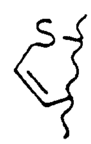

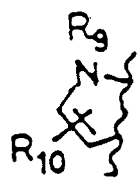

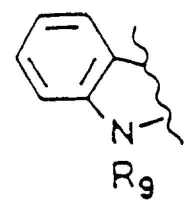

dans lesquels

$R_8$ est l'hydrogène ou méthoxy;

$R_6$ est méthoxy; hydroxy; amino; ou méthanesulfonylamino;

$R_7$ est l'hydrogène; méthoxy; ou hydroxy;

$R_9$ est méthyle;

$R_{10}$ est 2-phényl-2-éthoxycarbonylacétyle.

**3.** Utilisation d'un composé de formule I ou II selon la revendication 1 ou 2, où

A représente le reste

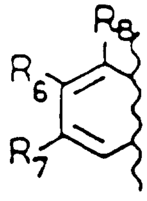

**4.** Utilisation d'un compose de formule I ou II selon l'une des revendications 1 à 3, où $R_8$ est l'hydrogène et $R_6$ et $R_7$ sont, indépendamment l'un de l'autre, hydroxy ou méthoxy, ou ensemble forment $-O-CH_2-O-$.

5. Utilisation d'un composé selon l'une des revendications 1 ou 2, où X est $-NHR_2$ ou $-NR_3R_4$.

6. Utilisation d'un composé selon la revendication 5, où X est $-NHR_2$, où $R_2$ est alkyle en $C_1$-$C_6$.

7. Utilisation d'un composé selon la revendication 6 où $R_2$ est alkyle en $C_1$-$C_4$ qui n'est pas substitué ou qui est substitué par hydroxy, phényle (qui peut être substitué par hydroxy, méthoxy ou $-O-CH_2-O-$) ou morpholino.

8. Dihydroisoquinoléines de formule

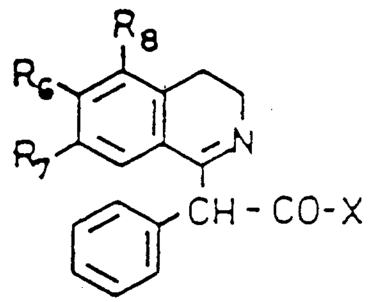

Ia

dihydrothiéno[3,2-c]pyridines de formule

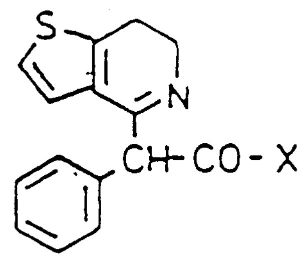

Ib

dihydropyrrolo[3,2-c]pyridines de formule

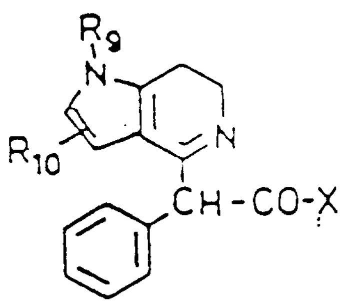

Ic

et dihydropyrido[3,4-b]indoles de formule

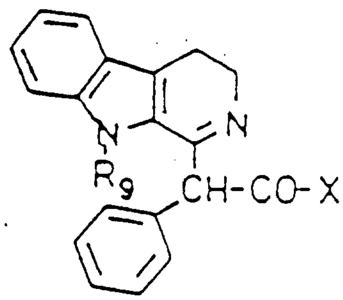

Id

ainsi que leurs formes tautomères avec leurs formes isomères E/Z et leurs formes énantiomères, où

X est $OR_1$; $NHR_2$; $NR_3R_4$, avec la condition que, dans les composés de formule Ia, X ne soit que $OR_1$, et

$R_1$ est l'hydrogène, alkyle en $C_1$-$C_4$; alcoxyalkyle en $C_1$-$C_4$;

$R_2$ est l'hydrogène; alcényle en $C_3$-$C_6$; alcynyle en $C_3$-$C_6$; cycloalkyle en $C_3$-$C_6$; cycloalcényle en $C_3$-$C_6$; alkyle en $C_1$-$C_6$ linéaire ou ramifié qui est éventuellement substitué une ou plusieurs fois par les substituants, des groupes a) à c), cités ci-dessous, et qui peuvent être identiques ou différents:

a) halogène; cyano; hydroxy; mercapto; alcoxy en $C_1$-$C_4$; alkylthio en $C_1$-$C_4$; amino; mono(alkyl en $C_1$-$C_4$)amino; di(alkyl en $C_1$-$C_4$)amino (où les radicaux alkyle peuvent être identiques ou différents);

b) phényle, éventuellement substitué une ou plusieurs fois de façon identique ou différente par les groupes halogène, alcoxy en $C_1$-$C_4$, hydroxy, mercapto, alkylthio en $C_1$-$C_4$, alkyle en $C_1$-$C_4$, amino, mono(alkyl en $C_1$-$C_4$)amino, di(alkyl en $C_1$-$C_4$)amino (les restes alkyle pouvant être identiques ou différents), acylamino en $C_2$-$C_3$, acyloxy en $C_2$-$C_3$, et le groupe $-O-(CH_2)_n-O-$ (avec n = 1 ou 2) lié au système du phényle en des positions adjacentes;

c) un hétérocycle monocyclique de 5 ou 6 chaînons saturé ou totalement ou partiellement insaturé, ayant jusqu'à 3 hétéroatomes du groupe N, O, S; et l'indole comme hétérocycle bicyclique (les hétérocycles précités pouvant être substitués une ou plusieurs fois par alkyle en $C_1$-$C_4$, cycloalkyle en $C_3$-$C_6$; cycloalcényle en $C_5$ ou $C_6$; acyle en $C_2$-$C_3$; alkylsulfonyle en $C_1$-$C_4$; ou phényle (qui peut à son tour être substitué jusqu'à trois fois de la manière décrite sous b));

$R_3$ et $R_4$, indépendamment l'un de l'autre, sont alkyle en $C_1$-$C_4$, qui peut éventuellement être substitué par phényle, le substituant phényle pouvant à son tour être substitué comme sous b) ci-dessus; ou bien

$R_3$ et $R_4$ forment ensemble avec l'atome d'azote auquel ils sont liés un noyau hétérocyclique de 5 ou 6 chaînons, totalement ou partiellement saturé (qui peut contenir en outre jusqu'à 2 autres hétéroatomes du groupe N, O, S), l'hétérocycle ainsi obtenu pouvant être substitué par alkyle en $C_1$-$C_4$, hydroxy, ou $(CH_2)_p$-$R_5$ (avec p = 0 ou 1); et

$R_5$ représente un radical phényle qui est éventuellement substitué comme sous b) ci-dessus;

$R_8$ est l'hydrogène; alkyle en $C_1$-$C_4$; ou alcoxy en $C_1$-$C_4$;

$R_6$ et $R_7$, qui peuvent être identiques ou différents, sont l'hydrogène; hydroxy; alkyle en $C_1$-$C_4$; alcoxy en $C_1$-$C_4$; amino; méthanesulfonylamino; ou bien

$R_6$ et $R_7$ forment ensemble $-O-(CH_2)_n-O-$ (avec n = 1, 2)

$R_9$ est l'hydrogène; alkyle en $C_1$-$C_4$;

$R_{10}$ est l'hydrogène; ou 2-phényl-2-éthoxycarbonylacétyle.

**9.** Procédé de préparation de composés de formule Ia à Id et de leurs formes tautomères selon la revendication 8

a) par cyclisation d'un amide d'acide phénylmalonique de formule générale III

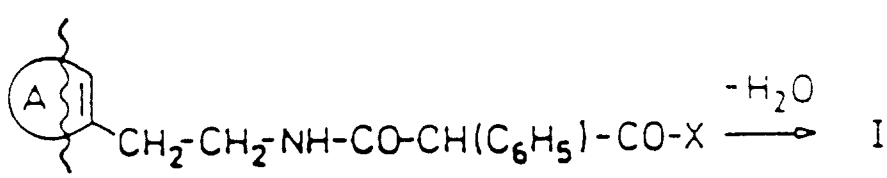

III

en présence d'un acide de Lewis comme agent de condensation, ou bien

b) à partir d'acides carboxyliques ou d'halogénures d'acides carboxyliques de formule IV où A est tel que défini ci-dessus et Y est OH ou halogène, par réaction avec des alcools ou des amines de formule générale VI, où X est tel que défini ci-dessus,

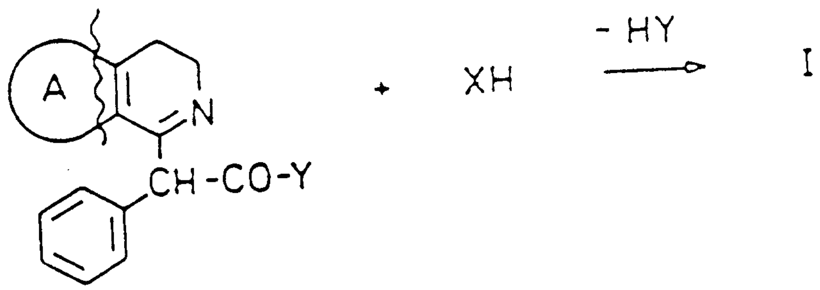


IV VI

en présence d'un carbodiimide approprié ou de carbonyldiimidazole comme agent de condensation.

**10.** Acides dihydropyridinylacétiques de formule

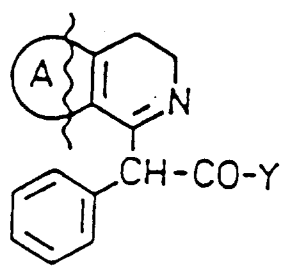

IV

ainsi que leurs formes tautomères

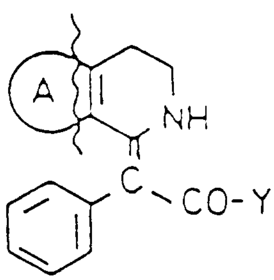

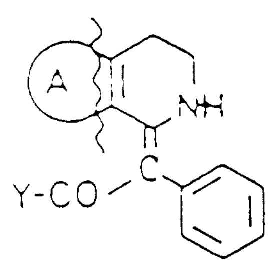

V' V"

V

où Y est OH ou halogène et

A représente les systèmes cycliques condensés

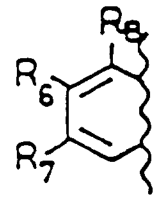

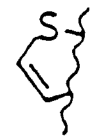

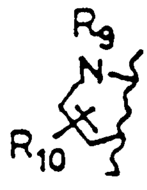

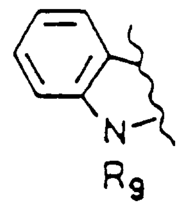

dans lesquels

$R_8$ est l'hydrogène; alkyle en $C_1$-$C_4$; ou alcoxy en $C_1$-$C_4$;

$R_6$ et $R_7$, qui peuvent être identiques ou différents, sont l'hydrogène; hydroxy; alkyle en $C_1$-$C_4$; alcoxy en $C_1$-$C_4$; amino; méthanesulfonylamino; ou bien

$R_6$ et $R_7$ forment ensemble $-O-(CH_2)_n-O-$ (avec n = 1, 2)

$R_9$ est l'hydrogène; alkyle en $C_1$-$C_4$;

$R_{10}$ est l'hydrogène; ou 2-phényl-2-éthoxycarbonylacétyle;

et leurs sels avec des acides et des bases.

**11.** Composé de formule générale IV ou Y selon la revendication 10, où Y est le fluor, le chlore ou le brome.

**12.** Acides phénylmaloniques de formule III

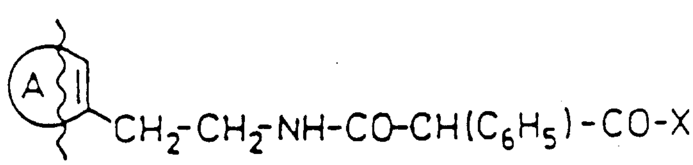

III

où X représente $OR_1$, $NHR_2$, $NR_3R_4$

et

$R_1$ est l'hydrogène, alkyle en $C_1$-$C_4$; alcoxyalkyle ayant jusqu'à 4 atomes de carbone;

$R_2$ est l'hydrogène, alcényle en $C_3$-$C_6$; alcynyle en $C_3$-$C_6$; cycloalkyle en $C_3$-$C_6$; cycloalcényle en $C_3$-$C_6$; alkyle en $C_1$-$C_6$ qui est éventuellement substitué une ou plusieurs fois par les substituants des groupes a) à c), cités ci-dessous, et qui peuvent être identiques ou différents:

a) halogène; cyano; hydroxy; mercapto; alcoxy en $C_1$-$C_4$; alkylthio en $C_1$-$C_4$; amino; mono(alkyl en $C_1$-$C_4$)amino; di(alkyl en $C_1$-$C_4$)amino (où les radicaux alkyle peuvent être identiques ou différents);

b) phényle, éventuellement substitué une ou plusieurs fois de façon identique ou différente par les groupes halogène, alcoxy en $C_1$-$C_4$, hydroxy, mercapto, alkylthio en $C_1$-$C_4$, alkyle en $C_1$-$C_4$, amino, mono(alkyl en $C_1$-$C_4$)amino, di(alkyl en $C_1$-$C_4$)amino (les restes alkyle pouvant être identiques ou différents), acylamino en $C_2$-$C_3$, acyloxy en $C_2$-$C_3$, et le groupe $-O-(CH_2)_n-O-$ (avec n = 1 ou 2) lié au système du phényle en des positions adjacentes;

c) un hétérocycle monocyclique de 5 ou 6 chaînons saturé ou totalement ou partiellement insaturé, ayant jusqu'à 3 hétéroatomes du groupe N, O, S; et l'indole comme hétérocycle bicyclique (les hétérocycles précités pouvant être substitués une ou plusieurs fois par alkyle en $C_1$-$C_4$, cycloalkyle en $C_3$-$C_6$; cycloalcényle en $C_5$ ou $C_6$; acyle en $C_2$-$C_3$; alkylsulfonyle en $C_1$-$C_4$; ou phényle (qui peut à son tour être substitué jusqu'à trois fois de la manière décrite sous b));

$R_3$ et $R_4$, indépendamment l'un de l'autre, sont alkyle en $C_1$-$C_4$, qui peut éventuellement être substitué par phényle, le substituant phényle pouvant à son tour être substitué comme sous b) ci-dessus; ou bien

$R_3$ et $R_4$ forment ensemble avec l'atome d'azote auquel ils sont liés un noyau hétérocyclique de 5 ou 6 chaînons, totalement ou partiellement saturé (qui peut contenir en outre jusqu'à 2 autres hétéroatomes du groupe N, O, S), l'hétérocycle ainsi obtenu pouvant être substitué par alkyle en $C_1$-$C_4$, hydroxy, ou $(CH_2)_p$-$R_5$ (avec $p = 0$ ou 1); et

$R_5$ représente un radical phényle qui est éventuellement substitué comme sous b) ci-dessus;

A représente les systèmes cycliques condensés

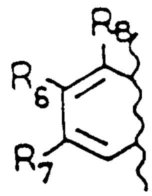

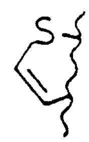

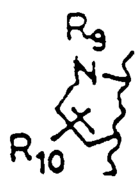

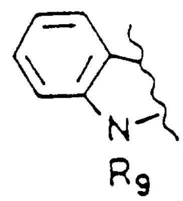

dans lesquels

$R_8$ est l'hydrogène; alkyle en $C_1$-$C_4$; ou alcoxy en $C_1$-$C_4$;

$R_6$ et $R_7$, qui peuvent être identiques ou différents, sont l'hydrogène; hydroxy; alkyle en $C_1$-$C_4$; alcoxy en $C_1$-$C_4$; amino; méthanesulfonylamino; ou bien

$R_6$ et $R_7$ forment ensemble $-O-(CH_2)_n-O-$ (avec $n = 1, 2$)

$R_9$ est l'hydrogène; alkyle en $C_1$-$C_4$;

$R_{10}$ est l'hydrogène; ou 2-phényl-2-éthoxycarbonylacétyle;

avec la condition que, dans les composés où A représente le système cyclique

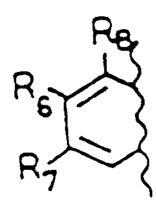

X ne soit que $OR_1$.